(19) Europäisches Patentamt
European Patent Office
Office européen des brevets

(11) **EP 4 521 925 B1**

(12) **EUROPÄISCHE PATENTSCHRIFT**

(45) Veröffentlichungstag und Bekanntmachung des Hinweises auf die Patenterteilung:
**08.04.2026   Patentblatt 2026/15**

(21) Anmeldenummer: **23745110.9**

(22) Anmeldetag: **14.07.2023**

(51) Internationale Patentklassifikation (IPC):
*A01N 37/44* (2006.01)   *A61L 15/26* (2006.01)
*A61L 15/42* (2006.01)   *A61L 15/46* (2006.01)
*A61L 15/60* (2006.01)   *A61P 17/02* (2006.01)

(52) Gemeinsame Patentklassifikation (CPC):
(C-Sets verfügbar)
**A61L 15/60; A61L 15/26; A61L 15/425; A61L 15/46; A61P 17/02;** A01N 25/04; A01N 59/16; A61L 2300/102                    (Forts.)

(86) Internationale Anmeldenummer:
**PCT/EP2023/069726**

(87) Internationale Veröffentlichungsnummer:
**WO 2024/017804 (25.01.2024 Gazette 2024/04)**

(54) **HYDROGEL ZUR REDUKTION VON BIOFILMEN**

HYDROGEL FOR THE REDUCTION OF BIOFILMS

HYDROGEL POUR LA RÉDUCTION DE BIOFILMS

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **18.07.2022   DE 102022117861**

(43) Veröffentlichungstag der Anmeldung:
**19.03.2025   Patentblatt 2025/12**

(73) Patentinhaber: **Paul Hartmann AG**
**89522 Heidenheim (DE)**

(72) Erfinder:
• **KETTEL, Markus**
  **89520 Heidenheim (DE)**
• **JANSSEN, Alena**
  **97209 Veitshöchheim (DE)**
• **NOSWORTHY, Jonathan**
  **89311 Neu-Ulm (DE)**

(74) Vertreter: **Paul Hartmann AG**
**Patents & Licensing**
**Paul-Hartmann-Straße 12**
**89522 Heidenheim (DE)**

(56) Entgegenhaltungen:
**WO-A1-2017/191453**

(52) Gemeinsame Patentklassifikation (CPC): (Forts.)

C-Sets
**A61L 15/26, C08L 75/04;**
A01N 25/04, A01N 59/16;
A01N 59/16, A01N 59/16

**Beschreibung**

Gebiet der Erfindung

Die vorliegende Erfindung betrifft ein Hydrogel zur Reduktion von Biofilmen enthaltend einen Wirkstoff, der in das Hydrogel eingebettet ist und das sich durch eine hohe Feuchtigkeits- und Wirkstoffabgabe auszeichnet. Das Hydrogel ist geeignet zur medizinischen Anwendung. Die Erfindung betrifft weiterhin ein Verfahren zur Herstellung des Hydrogels, die Verwendung des Hydrogels zu medizinischen Zwecken und eine Wundauflage, welche das Hydrogel als Wundkontaktschicht enthält.

Hintergrund und Aufgabe der Erfindung

Im Bereich der Wundversorgung ist allgemein bekannt, dass eine feuchte Wundumgebung die Heilung fördert. Ursächlich hierfür ist eine durch die feuchte Wundumgebung beschleunigte Epithelisierung, verringerte Narbenbildung und herabgesetzte Entzündungsreaktion. Die feuchte Wundversorgung eignet sich insbesondere für schlecht heilende, chronische und infizierte Wunden. Gerade bei Letzteren kann es durch Besiedlung der Wunde mit Mikroorganismen zu Komplikationen kommen. In der Regel geht eine Infektion der Wunde auf Bakterien zurück. Maßnahmen zur Entfernung bzw. Abtötung der Bakterien sind nicht immer erfolgreich, da die Bakterien sich häufig den geänderten Bedingungen anpassen. Dies umfasst insbesondere die Ausbildung von Resistenzen und den Zusammenschluss zu Biofilmen. Während die moderne Wundversorgung auf die Ausbildung von Resistenzen bereits reagiert hat und die topische Anwendung klassischer Antibiotika zurückgegangen ist, stellen Biofilme in Wunden immer noch ein ernsthaftes Problem dar und sind Gegenstand aktueller Forschung. WO2017191453 beschreibt die Anwendung von Metallkomplexen zur Bekämpfung von Biofilmen in Wunden. Darüber hinaus offenbart werden Hydrogele enthaltend derartige Metallkomplexe. Besagte Hydrogele wurden aus einer bestimmten Polyacrylsäure - Markenname Carbopol® - hergestellt. Obwohl Carbopol® ein beliebter Rohstoff in der Polymerchemie ist, geht er in der praktischen Anwendung mit Nachteilen einher. So führt die Verwendung von Carbopol® zu Staubbildung. Auch lässt sich das Pulver aufgrund seiner geringen Dichte nur schwer durch Rühren in Lösung bringen. Dies führt zu langen Mischzeiten, bei welchen es zu ungewollter Schaumbildung kommen kann. Ferner kann es bei (beabsichtigtem oder unbeabsichtigtem) Kontakt mit Feuchtigkeit zur Ausbildung von Klumpen kommen.

Im Tiermodell hat Carbopol® hemmende Effekte auf die Wundheilung gezeigt (Grip, Jostein, et al. "Sprayable Carbopol hydrogel with soluble beta-1, 3/1, 6-glucan as an active ingredient for wound healing-development and in-vivo evaluation." European Journal of Pharmaceutical Sciences 107 (2017): 24-31.). Somit besteht ein Bedarf an Hydrogelen, welche Wirkstoffe gegen Biofilme enthalten, diese zusammen mit Feuchtigkeit in hohem Maße in die Wunde abgeben und deren Substrat die Wundheilung nicht behindert. Es ist Aufgabe der vorliegenden Erfindung diesen Bedarf zu decken. Die Aufgabe wird mit einem Verfahren nach Anspruch 1, einem Hydrogel nach Anspruch 9 und einer Wundauflage nach Anspruch 15 gelöst.

Definitionen

Der Begriff Hydrogel bezeichnet im Rahmen der vorliegenden Erfindung ein feindisperses System aus mindestens einer festen und einer flüssigen Phase. Diese feste Phase bildet ein schwammartiges, dreidimensionales Netzwerk, dessen Poren durch eine Flüssigkeit (Lyogel) ausgefüllt sind. Beide Phasen durchdringen sich. Bevorzugt durchdringen sich beide Phasen vollständig. Durch Wasseraufnahme kann das dreidimensionale Netzwerk durch Quellen sein Volumen vergrößern, ohne dabei den strukturellen Zusammenhalt zu verlieren. Der Begriff Hydrogel wird im Folgenden auch synonym als Hydrogelzusammensetzung oder Hydrogelmatrix bezeichnet. Durch Variation der Reaktionsparameter des erfindungsgemäßen Herstellungsverfahrens kann das resultierende Hydrogel mitunter einen schaumartigen Charakter erhalten. Auch derartige Produkte werden im Folgenden als erfindungsgemäße Hydrogele bezeichnet. Entscheidend ist, dass sie durch das erfindungsgemäße Herstellungsverfahren erhalten werden können.

Der Begriff Wirkstoff kann im Rahmen der vorliegenden Erfindung die folgenden Bedeutungen haben:

a) die Komplexverbindung $Ag_2Zn(EDTA)$,
b) eine wässrige Lösung der besagten Komplexverbindung oder
c) eine wässrige Lösung der Ausgangssubstanzen Silbernitrat, Zinksulfat bzw.

Zinksulfatmonohydrat und Tetrasodium EDTA, die sich zu dem besagten Komplex verbinden. Sofern nicht anders ausgeführt, kann unter dem Begriff "Wirkstoff" primär die unter a) genannte Komplexverbindung verstanden werden. Der Wirkstoff hat sowohl Biofilm reduzierende als auch antibakterielle Eigenschaften und fördert zudem die Freisetzung von Feuchtigkeit aus den erfindungsgemäßen Hydrogelen.

Der Begriff Reaktionsmischung bedeutet eine Mischung aller Bestandteile für den Ablauf des erfindungsgemäßen Verfahrens zur Herstellung eines Biofilm reduzierenden Hydrogels. Dies schließt sämtliche Reaktanden mit ein und kann auch optionale Hilfsstoffe wie Konsistenzgeber, Stabilisatoren, Säuren, Laugen etc. beinhalten. Im Folgenden werden verschiedene Ausführungsformen für das erfindungsgemäße Verfahren erläutert. Die Zusammensetzung der Reaktionsmischung kann von der jeweiligen Ausführungsform abhängen.

Der Begriff Biofilm meint einen dünnen, flächig ausgebreiteten Schleimfilm, in dem Populationen von Mikroorganismen vorliegen. Der Schleimfilm wird von den Mikroorganismen gebildet und ist eine die Mikroorganismen einschließende Matrix aus extrazellulärem polymerem Material. Typischerweise handelt es sich bei den Populationen von Mikroorganismen um Mischpopulationen. Biofilme bilden sich auf Oberflächen aus, zu denen auch Wundgewebe gehören kann. Die im Biofilm organisierten Mikroorganismen zeigen eine erhöhte Widerstandskraft gegen herkömmliche Antibiotika, Desinfektionsmittel und gegenüber dem Immunsystem höher entwickelter Lebewesen.

Der Begriff "Biofilm reduzierend" umfasst sowohl die Abtötung von Bakterien (desinfizierende Eigenschaften) als auch das Aufbrechen von chemischen Bindungen und den Entzug chemischer Bindungspartner aus der Biofilm bildenden extrazellulären Matrix. Letzteres setzt die Abwehrkraft der zuvor im Biofilm organisierten Mikroorganismen gegenüber Einflüssen wie Desinfektionsmitteln, Antibiotika, dem Immunsystem oder Umwelteinflüssen allgemein herab. Als Biofilm reduzierend werden im Rahmen der vorliegenden Erfindung nur derartige Substanzen verstanden, die geeignet sind, auf offene Wunden aufgebracht zu werden, ohne eine nennenswerte Schadwirkung am zu behandelnden Individuum zu entfalten.

Der Begriff Ausgangslösung meint eine Lösung, die zumindest Wasser, ein Amin-terminiertes Präpolymer und den Wirkstoff enthält. Weitere Bestandteile wie z.B. ein mehrwertiger Alkohol, bevorzugt Glycerin, oder eine Base, bevorzugt Ammoniak, können optional enthalten sein.

Die Begriffe Feuchtigkeitsabgabe und Feuchtigkeitsfreisetzung meinen dasselbe.

Der Ausdruck Kolonie bildenden Einheit (CFU) meint eine einzelne teilungsfähige Zelle eines Einzellers, insbesondere eines humanpathogenen Einzellers oder Bakteriums.

Beschreibung der Erfindung

Die erfindungsgemäßen Hydrogele haben hervorragende Eigenschaften hinsichtlich ihrer Fähigkeit Wasser zu speichern und an eine Wunde abzugeben. Es hat sich gezeigt, dass der Wirkstoffgehalt im Hydrogel, also die Konzentration des Komplexes im Hydrogel, einen Einfluss darauf hat, in welchem Ausmaß Feuchtigkeit an die Wunde durch das Hydrogel abgegeben wird. Mit zunehmender Konzentration an Wirkstoff im Hydrogel nimmt die Feuchtigkeitsabgabe zu.

Die durch den Wirkstoff verstärkt aus dem erfindungsgemäßen Hydrogel freigesetzte Feuchtigkeit hat den Vorteil, dass zusammen mit der Feuchtigkeit Wirkstoff in die Wunde abgegeben wird.

Überraschenderweise haben Messungen gezeigt, dass die Adhäsionskraft des erfindungsgemäßen Hydrogels durch die Einbettung des Wirkstoffs gesteigert werden kann. Gleichzeitig bleibt der atraumatische Charakter des Hydrogels als Wundkontaktschicht erhalten, da das Hydrogel nicht mit der Wunde verklebt. Die Einbettung des Wirkstoffs in das Hydrogel kann es damit unter Umständen erlauben weitere Klebeschichten bzw. einen umlaufenden Kleberand kleiner zu gestalten bzw. - in Abhängigkeit vom Einsatzzweck der Wundauflage - ganz auf diese zu verzichten. Dies erleichtert die Herstellung einer solchen erfindungsgemäßen Wundauflage und spart Kosten. Während der Entwicklung der vorliegenden Erfindung hat sich gezeigt, dass mit steigendem Anteil des Wirkstoffs in dem Hydrogel die Adhäsionskraft des Hydrogels zunimmt, d.h. das Hydrogel haftet umso stärker an der Haut bzw. Wunde je mehr $Ag_2Zn(EDTA)$ darin enthalten ist. Dies wird zumindest teilweise darauf zurückgeführt, dass mit zunehmendem Wirkstoffanteil der Grad an Quervernetzung innerhalb der Hydrogelmatrix zurückgeht. In diesem Sinne umfasst die Erfindung auch die Verwendung eines Komplexes $Ag_2Zn(EDTA)$ in einem Hydrogel, um die Adhäsionskraft des Hydrogels einzustellen oder zu steigern. Insbesondere kann die Verwendung dazu genutzt werden die Adhäsionskraft des Hydrogels auf Gewebe wie Haut oder Wundgewebe einzustellen.

Das erfindungsgemäße Verfahren zur Herstellung eines Hydrogels umfasst die folgenden Schritte:

i. Bereitstellen eines Isocyanat-terminierten Präpolymers enthaltend Polyalkylenoxideinheiten,

ii. Bereitstellen eines Amin-terminierten Präpolymers enthaltend Polyalkylenoxideinheiten,

iii. Lösen der unter ii bereitgestellten Substanz in einer Wasser enthaltenden Flüssigkeit, um eine wässrige Zubereitung zu erhalten,

iv. Mischen der wässrigen Zubereitung und einer Lösung, insbesondere einer wässrigen Lösung, enthaltend einen Komplex $Ag_2Zn(EDTA)$, um eine Ausgangslösung zu erhalten,

v. Zusammenführen der Ausgangslösung und des Isocyanat-terminierten Präpolymers zu einer Reaktionsmischung, wodurch diese durch Polymerisation zum Hydrogel umgesetzt werden,

wobei der pH-Wert der Lösung aus Schritt iv mindestens 9, bevorzugt 10 bis 12, ist und wobei die Reaktionsmischung aus Schritt v keine Acrylsäure oder Polyacrylsäure enthält.

Beim letzten Schritt v werden das Isocyanat-terminierte Präpolymer aus Schritt i und das in der Ausgangslösung enthaltene Amin-terminierte Präpolymer aus Schritt ii miteinander zur Reaktion gebracht. Weitere Reaktanden wie ein mehrfacher Alkohol können ebenfalls vorhanden sein. Während der Reaktion findet eine Polymerisation statt, das heißt, dass die beiden Präpolymere unter Ablauf einer Polymerisation eine quervernetzte Gelmatrix ausbilden. Wasser und der Komplex $Ag_2Zn(EDTA)$ werden dabei in die Gelmatrix aufgenommen, können jedoch aus dieser wieder freigesetzt werden. Als Ergebnis der Reaktion werden die erfindungsgemäßen, wirkstoffhaltigen Hydrogele erhalten.

Die Ausgangslösung und das Isocyanat-terminierte Präpolymer können sukzessiv - das heißt nach und nach - zusammengeführt werden. Es ist möglich die Ausgangslösung und das Isocyanat-terminierte Präpolymer in einer Gussform zusammenzuführen. Nach Abschluss der Reaktion kann das fertige Hydrogel aus der Gussform entnommen werden und die durch die Gussform vorgegebene Form beibehalten.

Die Komplexverbindung $Ag_2Zn(EDTA)$ stellt den Biofilm reduzierenden Wirkstoff innerhalb des Hydrogels dar und ist erhältlich durch Lösen der folgenden Verbindungen in einer polaren, vorzugsweise wässrigen Flüssigkeit, insbesondere Wasser: Tetrasodium EDTA (auch bekannt als Tetranatrium-EDTA), Silbernitrat und Zinksulfat bzw.

Zinksulfatmonohydrat. Für die Darstellung von $Ag_2Zn(EDTA)$ ist es möglich zunächst polare, bevorzugt wässrige Lösungen von Tetrasodium EDTA (z.B. eine 3,8 %ige Lösung) und Silbernitrat (z.B. eine 6,8 %ige Lösung) miteinander reagieren zu lassen und den ausgefallenen Feststoff herauszufiltern. Das Filtrat kann einer polaren, bevorzugt wässrigen Lösung von Zinksulfatmonohydrat (z.B. eine 1,9 %ige Lösung) beigemengt werden. Nach einer weiteren Filtrierung wird der Komplex $Ag_2Zn(EDTA)$ erhalten.

Das erfindungsgemäße Verfahren und die damit einhergehenden chemischen Reaktionen können bei Raumtemperatur ablaufen. Bei der Zugabe von Alkoholen zur Reaktionsmischung kann eine niedrigere Temperatur mitunter zu besseren Ergebnissen führen, was an anderer Stelle näher erläutert wird.

Die Reaktionsmischung des erfindungsgemäßen Verfahrens und das erfindungsgemäße Hydrogel enthalten keine Acrylsäure bzw. Polyacrylsäure. Von der Beimengung von Acrylsäure wird abgeraten, da diese einen negativen Einfluss auf die Wundheilung ausüben könnte.

Beim erfindungsgemäßen Herstellungsverfahren kann das Massenverhältnis des Isocyanat-terminierten Präpolymers zum Amin-terminierten Präpolymer zwischen 1,3 und 3,2 liegen, bevorzugt zwischen 1,4 und 1,8, besonders bevorzugt zwischen 1,5 und 1,7 und ganz besonders bevorzugt zwischen 1,55 und 1,65.

Bei den genannten Hydrogelen muss die feste Phase nicht zwangsläufig allein durch ein Polymer gebildet werden, das aus der Reaktion zwischen einem Amin-terminierten Präpolymer und einem Isocyanat-terminierten Präpolymer entsteht. An der Reaktion kann ebenfalls ein mehrwertiger Alkohol beteiligt sein, dessen freie Hydroxygruppen mit Isocyanatgruppen reagieren können. Die Komponente des mehrwertigen Alkohols trägt dabei zu einer zusätzlichen Vernetzung bei, die insbesondere bei mehrwertigen Alkoholen mit mehr als zwei Hydroxygruppen zu einer dreidimensionalen Vernetzung der Präpolymere führt. In der Reaktion zwischen einem mehrwertigen Alkohol und einer Isocyanatgruppe entsteht ein Carbamidsäureester, der auch als Urethan bezeichnet wird. Diese Reaktion kann durch Säuren oder Basen als Katalysator beschleunigt werden und unter Zufuhr von thermischer Energie rückgängig gemacht werden. Es ist möglich das erfindungsgemäße Verfahren bei Raumtemperatur auszuführen. Wird die Reaktionstemperatur konstant zwischen 5°C und 30°C, bevorzugt zwischen 5°C und 20°C, gehalten, kann diese Reaktion in einem ausreichenden Verhältnis erfolgen, so dass Hydrogele mit kovalent eingebundenen mehrwertigen Alkoholen erhalten werden, die vorteilhafte Eigenschaften aufweisen. In diesem Sinne kann die Reaktionsmischung mindestens einen mehrwertigen Alkohol enthalten. Dieser kann ausgewählt werden aus der Gruppe der zweiwertigen, dreiwertigen, vierwertigen, fünfwertigen oder sechswertigen Alkohole. Insbesondere kann der Alkohol gewählt werden aus der Gruppe der Glykole, insbesondere Ethylenglykol, Polyethylenglykole mit einer Masse von 200 g/mol bis 6000 g/mol, bevorzugt Polyethylenglykole mit einer Masse von 300 g/mol bis 2000 g/mol, sowie Sorbitol oder Glycerin oder Mischungen hiervon. Diese Alkohole sind daneben hervorragend als Feuchtigkeitsspender geeignet und stellen somit für die die Wunde umgebende Haut eine pflegende Komponente dar. Hydrogele, die einen oder mehrere dieser Alkohole als Partner in der oben beschriebenen Reaktion enthalten, weisen ein hohes Absorptionsvermögen für Wundexsudat und einen verringerten Feuchtigkeitsverlust auf. Sie weisen eine Adhäsionskraft auf, die einen atraumatischen Verbandwechsel erlaubt. Aufgrund ihrer geringen Zytotoxizität sind sie sehr gut verträglich für Wundgewebe. Außerdem sind derartige Gele in der Lage, für die Wundheilung notwendige Wachstumsfaktoren des Wundexsudats aufzukonzentrieren und so die Wundheilung zu beschleunigen. Die Anwesenheit von Mehrfachalkohol in der Wunde führt zu einer verlangsamten Verdunstung der Feuchtigkeit, wodurch die Wunde länger feucht gehalten wird.

Deswegen wird gemäß einer bevorzugten Ausführungsform des erfindungsgemäßen Verfahren der Reaktionsmischung Glycerin beigemengt. Das Glycerin kann der Wasser enthaltenden Flüssigkeit aus Schritt iii des Verfahrens zugesetzt werden. Demgemäß zeichnet sich das Verfahren bevorzugt dadurch aus, dass die Wasser enthaltende Flüssigkeit in Schritt iii Glycerin enthält.

Die Menge des zugesetzten Glycerins wird vorzugsweise so gewählt, dass die Reaktionsmischung in Schritt v des Verfahrens 10 bis 30 Gew.-% Glycerin enthält.

Die so hergestellten Hydrogele weisen besonders vorteilhafte Eigenschaften hinsichtlich der Zellkompatibilität, des Flüssigkeitsverlusts und des Haftvermögens auf.

In einer besonders bevorzugten Ausführungsform wird als mehrwertiger Alkohol Glycerin in einer Konzentration von 15-25 Gew.-% verwendet. Die so hergestellten Hydrogele weisen außerdem ein besonders hohes Absorptionsvermögen auf.

Gemäß einer anderen Ausführungsform wird als mehrwertiger Alkohol Ethylenglykol in einer Konzentration von 5-30 Gew-%, bevorzugt 10-25 Gew-%, besonders bevorzugt 15-20 Gew.- % verwendet. Derartige Hydrogele weisen vorteilhafte Eigenschaften hinsichtlich des Feuchtigkeitsverlustes, der Absorptionsfähigkeit und der Zellkompatibilität auf.

Gemäß einer weiteren Ausführungsform wird als mehrwertiger Alkohol Sorbitol in einer Konzentration von 5-30 Gew-%, bevorzugt 10-25 Gew-%, besonders bevorzugt 15-20 Gew.- % verwendet. Derartige Hydrogele weisen vorteilhafte Eigenschaften hinsichtlich der Absorptionsfähigkeit und der Zellkompatibilität auf.

Gemäß einer weiteren Ausführungsform wird als mehrwertiger Alkohol PEG300 (Polyethylenglycol mit einer mittleren relativen Molekülmasse 300 g/mol) in einer Konzentration von 5-30 Gew-%, bevorzugt 10-25 Gew.-%, besonders bevorzugt 15-20 Gew.- % verwendet. Derartige Hydrogele weisen vorteilhafte Eigenschaften hinsichtlich des Feuchtigkeitsverlustes und der Absorptionsfähigkeit auf.

Gemäß einer weiteren Ausführungsform wird als mehrwertiger Alkohol PEG2000 (Polyethylenglycol mit einer mittleren relativen Molekülmasse 2000 g/mol) in einer Konzentration von 5-30 Gew-%, bevorzugt 10-25 Gew.-%, besonders bevorzugt 15-20 Gew.- % verwendet. Derartige erfindungsgemäße Hydrogele weisen vorteilhafte Eigenschaften hinsichtlich der Zellkompatibilität auf.

Im Rahmen des erfindungsgemäßen Verfahrens zur Herstellung eines Biofilm reduzierenden Hydrogels kann die Reaktionsmischung also einen Mehrfachalkohol, insbesondere Glycerin, enthalten. Die Reaktionsmischung kann bevorzugt 10 bis 30 Gew.-% Mehrfachalkohol wie Glycerin enthalten. Besonders bevorzugt enthält die Reaktionsmischung 15 bis 25 Gew.-% Mehrfachalkohol wie Glycerin. Ganz besonders bevorzugt enthält die Reaktionsmischung 18 bis 23 Gew.-% Mehrfachalkohol wie Glycerin. Der Begriff Mehrfachalkohol schließt auch eine Mischung verschiedener Mehrfachalkohole ein. Hierzu gehören neben Glycerin die bereits zuvor genannten Alkohole Ethylenglykol, Sorbitol, PEG300, PEG2000. Bevorzugt wird dabei einer der vorangegangenen Alkohole mit Glycerin kombiniert, so dass die beiden Alkohole 10 bis 30 Gew.-% der Reaktionsmischung im erfindungsgemäßen Verfahren ausmachen. Während der Entwicklung der vorliegenden Erfindung hat sich gezeigt, dass die wirkstoffhaltige Lösung, welche im Rahmen des erfindungsgemäßen Verfahrens zur Herstellung des Hydrogels verwendet wird, durch einen alkalischen pH-Wert stabilisiert werden kann. Bei neutralem pH-Wert und sauren pH-Werten kann es hingegen zu einer Präzipitation und dadurch zu einem Ausfallen der chemischen Komponenten aus der Lösung kommen. Die Wahrscheinlichkeit einer Präzipitation kann reduziert werden, wenn der pH-Wert der wirkstoffhaltigen Lösung auf einen pH-Wert von mindestens 9 eingestellt wird. Deswegen beträgt der pH-Wert der wirkstoffhaltigen Lösung erfindungsgemäß mindestens 9, bevorzugt 10 bis 12. Die Wahrscheinlichkeit einer Präzipitation ist darüber hinaus auch von der Konzentration des Wirkstoffes in der Lösung abhängig. Eine höhere Konzentration macht eine Präzipitation wahrscheinlicher. Bevorzugt wird ab einer Wirkstoffkonzentration von 1 % $Ag_2Zn(EDTA)$ ein pH-Wert der wirkstoffhaltigen Lösung von mindestens 9,5 eingestellt, ab einer Wirkstoffkonzentration von 1,5 % $Ag_2Zn(EDTA)$ ein pH-Wert von mindestens 10, ab einer Wirkstoffkonzentration von 2 % $Ag_2Zn(EDTA)$ ein pH-Wert von mindestens 10,5 und ab einer Wirkstoffkonzentration von 2,5 % $Ag_2Zn(EDTA)$ ein pH-Wert von mindestens 11.

Bevorzugt werden zum Einstellen des pH-Wertes Ammoniak und/oder Natriumhydroxid verwendet. Die Verwendung von Ammoniak ist bevorzugt, da hier die besten und stabilsten Hydrogele erhalten wurden. Es hat sich gezeigt, dass bei Verwendung von NaOH die Natrium-Ionen mit dem im $Ag_2Zn(EDTA)$ enthaltenen Silber konkurrieren. Bei Bedarf kann zum Absenken eines zu hohen pH-Wertes Essigsäure beigemengt werden.

Bevorzugt ist die wirkstoffhaltige Lösung eine wässrige Lösung. Das Wasser hat hierbei den Vorteil, dass es im Laufe der Reaktion in die Hydrogelmatrix eingelagert wird und anschließend an die Wunde abgegeben werden kann.

Es wird an dieser Stelle betont, dass der pH-Wert der wirkstoffhaltigen Lösung nicht dem pH-Wert der Reaktionsmischung oder des später erhaltenen erfindungsgemäßen Hydrogels entsprechen muss. Das Hydrogel kann auch einen neutralen oder leicht sauren pH-Wert aufweisen, ohne dass eine Präzipitation des enthaltenen Wirkstoffs stattfindet.

Generell ist aus dem Stand der Technik bekannt Wundauflagen zur feuchten Wundversorgung bereitzustellen, die eine isotone Lösung an die Wunde abgeben. Im Rahmen der hier vorliegenden Erfindung hat sich gezeigt, dass die meisten dieser Lösungen im Zusammenhang mit dem erfindungsgemäßen Hydrogel nachteilig sind. Der Grund liegt darin, dass diese Lösungen in der Regel Chlorid-Ionen enthalten. Wie herausgefunden wurde, gehen die Chlorid-Ionen eine Reaktion mit dem im $Ag_2Zn(EDTA)$ enthaltenen Silber ein und bilden schwer lösliches Silberchlorid. Gemäß einer bevorzugten Ausführungsform des erfindungsgemäßen Verfahrens enthält die Reaktionsmischung keine Chloridionen oder Chlorsalze - insbesondere kein NaCl. Entsprechend enthält dann auch das erfindungsgemäße Hydrogel bzw. das durch das erfindungsgemäße Verfahren erhältliche Hydrogel bevorzugt keine Chloridionen oder Chlorsalze.

Bevorzugt enthalten die Reaktionsmischung und / oder das erfindungsgemäße Hydrogel kein Triethanolamin. Von der Beimengung von Triethanolamin wird abgeraten, da sich dieses störend auf die Reaktion des erfindungsgemäßen Verfahrens auswirken könnte. Das Triethanolamin könnte mit dem in der Reaktionsmischung enthaltenen Amin-terminierten Präpolymer konkurrieren. Es wird deswegen auch davon abgeraten Triethanolamin zur Einstellung des pH-Wertes des Hydrogels oder der wirkstoffhaltigen Lösung zu verwenden.

Das erfindungsgemäße Verfahren ist typischerweise dadurch gekennzeichnet, dass das Amin-terminierte Präpolymer bei der Umsetzung in Verfahrensschritt v zumindest teilweise kovalent gebunden wird. Gemäß einer Ausführungsform des erfindungsgemäßen Verfahrens werden mindestens 70 Gew.-% des in der Reaktionsmischung eingesetzten Amin-terminierten Präpolymers im Rahmen des erfindungsgemäßen Herstellungsverfahrens kovalent gebunden, bevorzugt werden mindestens 80 Gew.-% des eingesetzten Amin-terminierten Präpolymers kovalent gebunden, besonders bevorzugt werden mindestens 90 Gew.-% des eingesetzten Amin-terminierten Präpolymers kovalent gebunden, ganz besonders bevorzugt werden mindestens 95 Gew.-% des in der Reaktionsmischung eingesetzten Amin-terminierten Präpolymers kovalent gebunden.

Die Reaktionsmischung kann 0,5 bis 4 Gew.-% des Komplexes $Ag_2Zn(EDTA)$ enthalten. Der Anteil des Komplexes in der Reaktionsmischung hat Einfluss auf die Menge des Wirkstoffs im resultierenden Hydrogel. Darüber hinaus beeinflussen unterschiedliche Mengen an Wirkstoff im Hydrogel die Wirksamkeit gegenüber Mikroorganismen, Biofilmen und die Freisetzung von Feuchtigkeit aus dem Hydrogel. Bevorzugt enthält die Reaktionsmischung 0,5 bis 3,5 Gew.-% des Komplexes $Ag_2Zn(EDTA)$. Besonders bevorzugt enthält die Reaktionsmischung 1 bis 3 Gew.-% des Komplexes $Ag_2Zn(EDTA)$. Ganz besonders bevorzugt enthält die Reaktionsmischung 1 bis 2 Gew.-% des Komplexes $Ag_2Zn(EDTA)$.

Gemäß einer bevorzugten Ausführungsform beträgt die Summe der Massen aus Amin-terminiertem Präpolymer und Isocyanat-terminiertem Präpolymer 10 bis 30 Gew.-% der Reaktionsmischung.

Die Reaktionsmischung kann das Isocyanat-terminierte Präpolymer in einer Menge von 10 - 25 Gew.-% enthalten. Bevorzugt enthält die Reaktionsmischung 11 bis 23 Gew.-%, besonders bevorzugt 12 bis 20 Gew.-%, ganz besonders bevorzugt 13 bis 18 Gew.-% des Isocyanat-terminierten Präpolymers.

Ferner kann die Reaktionsmischung das Amin-terminierte Präpolymer in einer Menge von 3 bis 15 Gew.-%, vorzugsweise 5 bis 15 Gew.-%, besonders bevorzugt 6 bis 12 Gew.-%, ganz besonders bevorzugt 7 bis 10 Gew.-% enthalten.

Die Polyalkylenoxideinheiten der beiden zuvor erwähnten Präpolymere können durch Polyethylenoxid- und/oder Polypropylenoxideinheiten gebildet werden, wobei das Gewichtsverhältnis von Ethylenoxid- zu Propylenoxideinheiten vorzugsweise 3 : 1 bis 7 : 1 beträgt. Darüber hinaus ist das Isocyanat-terminierte Präpolymer vorteilhafterweise mindestens dreiarmig verzweigt. Insbesondere ist das Isocyanat-terminierte Präpolymer genau dreiarmig verzweigt.

Ein mögliches Isocyanat-terminiertes Präpolymer mit aliphatischen Isocyanat-Gruppen ist beispielsweise ein dreiarmiges Copolymer aus Propylenglykol- und Ethylenglykoleinheiten, welches endständig jeweils mit einem Molekül Isophorondiisocyanat umgesetzt wurde. Es weist typischerweise einen Gehalt reaktiver Isocyanatendgruppen (NCO-Gruppen) von 3,0 % bis 3,4 %, bevorzugt 3,2 % auf und ein molares Verhältnis von Ethylenoxideinheiten zu Propylenoxideinheiten von 3 : 1 bis 4:1. Bei dem Isocyanat-terminierten Präpolymer kann es sich um Aquapol® handeln. Diese Chemikalie kann unter dem Handelsnamen Aquapol PI-13000-31 vom Hersteller Carpenter (Richmond, USA) bezogen werden.

Ein mögliches Amin-terminiertes Präpolymer ist beispielsweise ein Triblock-Polymer aus zwei Propylenglykol-, Ethylenglykol- und wieder Propylenglykoleinheiten, wobei das Polymer endständig jeweils mit 2-Aminopropylgruppen aminfunktionalisiert ist. Es weist üblicherweise einen Gehalt reaktiver Aminendgruppen von 0,9554 mmol/g bei einer Molekülmasse von durchschnittlich etwa 2000 g/mol und einer Dispersität von 1,08, gemessen durch Gelpermeationschromatographie, auf und ein molares Verhältnis von Ethyleneinheiten zu Propyleneinheiten von 3:1 bis 7:1, bevorzugt 39:6. Ein derartiges Amin-terminiertes Präpolymer kann unter dem Namen Jeffamin® ED-2003 vom Hersteller Huntsman (Everberg, Belgien) bezogen werden.

Weiterhin umfasst die Erfindung ein Hydrogel mit Biofilm reduzierenden Eigenschaften erhältlich oder erhalten durch das erfindungsgemäße Verfahren.

Die Hydrogele der vorliegenden Erfindung können als feste Phase ein Polymer mit Polyurethan- und Polyharnstoffgruppen enthalten. Als flüssige Phase können sie insbesondere Wasser und optional einen mehrwertigen Alkohol enthalten. Propylenglykol kann hiervon ausgenommen sein, da diese Substanz weniger zellkompatibel als beispielsweise Glycerol ist.

Die Hydrogele sind geeignet zur Behandlung von Wunden. Das Hydrogel kann auf unterschiedliche Arten zur Wundbehandlung eingesetzt werden. Das Gel kann zuerst auf die Wunde aufgetragen und dann optional mit einer Wundauflage abgedeckt werden. Die Wundauflage kann dabei das Hydrogel fixieren und vor äußeren Einflüssen abschirmen. Das Hydrogel kann darüber hinaus auch ohne Abdeckung auf der Wunde belassen werden. Dies ist vor allem bei kürzeren Applikationszeiten möglich und an Hautarealen, bei denen ein Verrutschen des Hydrogels unwahrscheinlich ist. Die Applikation ohne Abdeckung ermöglicht die besonders schnelle Inspektion der Wunde und des Heilungsverlaufs.

Als wasserhaltige Hydrogele können im Zusammenhang mit der vorliegenden Erfindung insbesondere Hydrogele verwendet werden, die eine zusammenhängende, diskrete Schicht bilden und unter einem Druck, der bei bestimmungsgemäßer Anwendung des Hydrogels auftritt, kein Wasser abgeben. Hierzu sollte der Wasseranteil in der Reaktionsmischung des Herstellungsverfahrens entsprechend eingestellt werden. In Abhängigkeit der Anteile der übrigen Reaktanden kann die Reaktionsmischung einen Wasseranteil von 40 Gew.-% oder mehr enthalten. Bevorzugt enthält die Reaktionsmischung einen Wasseranteil von 40 bis 65 Gew.-%, besonders bevorzugt 50 bis 65 Gew.-%, ganz besonders bevorzugt 50 bis 60-Gew.-%. Dementsprechend können erfindungsgemäße Hydrogele einen Wassergehalt von mindesten 40 Gew.-% haben, bevorzugt 40 bis 65 Gew.-%, besonders bevorzugt 50 bis 65 Gew.-% und ganz besonders bevorzugt 50 bis 60-Gew.-%.

Erfindungsgemäße Hydrogele haben in der Regel einen pH-Wert, der zwischen 6,5 und 9,5, bevorzugt zwischen 6,5 und 8, besonders bevorzugt zwischen 7 und 8, liegt und damit ein optimales Spektrum zur Behandlung infizierter Wunden abdeckt. Ein besonderer Vorteil der erfindungsgemäßen Hydrogele ist, dass die durch das erfindungsgemäße Herstellungsverfahren erhältlichen Hydrogele in der Regel ohne weiteres Zutun einen optimalen pH-Wert aufweisen.

Obwohl ein saurer pH-Wert häufig als optimal für die Förderung der Wundheilung angesehen wird, ist mittlerweile bekannt, dass dies nicht immer für infizierte Wunden gelten muss. In Abhängigkeit von den in der Wunde vorkommenden Erregern kann ein basischer pH des einzusetzenden Hydrogels bzw. der Wundauflage vorteilhaft sein. Sollte eine genaue Einstellung des pH-Wertes des erfindungsgemäßen Hydrogels gewünscht sein, so kann dies durch Beimengung geeigneter Substanzen wie Ammoniak oder Essigsäure erfolgen. Ein neutraler oder leicht saurer pH-Wert wird empfohlen zur Verhinderung von Infektionen oder bei lediglich geringer Besiedelung der Wunde. Ein alkalischer pH-Wert (pH > 7) wird empfohlen, wenn bereits eine signifikante Besiedelung der Wunde mit Erregern erfolgt ist. Es gibt Hinweise darauf, dass zahlreiche bakterielle Enzyme und Toxine in einem alkalischen Milieu gehemmt werden. Sollte eine Kombination der erfindungsgemäßen Wundauflage bzw. des Hydrogels mit Antibiotika angestrebt werden, wird ebenfalls ein alkalischer pH-Wert empfohlen, da die meisten Antibiotika durch ein saures Milieu gehemmt werden.

Das erfindungsgemäße Hydrogel kann Biofilme, welche grampositive und/oder gramnegative Bakterien enthalten, reduzieren. Insbesondere ist das Hydrogel geeignet, um Biofilme der grampositiven Erreger *Staphylococcus aureus* (*S. aureus*) und der gramnegativen Erreger *Pseudomonas aeruginosa* (*P. aeruginosa*) zu reduzieren. Gemäß einer bevorzugten Ausführungsform wird in einem Test nach ASTM E2871-13 oder ASTM E2871 - 21 eine Reduktion an Kolonie bildenden Einheiten (CFU) bzw. eine Reduktion der Anzahl der Erreger um mindestens $\log_{10} = 2$, besonders bevorzugt mindestens $\log_{10} = 3$, ganz besonders bevorzugt mindestens $\log_{10} = 5$, erzielt - insbesondere gegenüber *S. aureus* und/oder *P. aeruginosa.* Eine überraschend hohe Wirksamkeit wird erzielt bei der Reduktion von Biofilmen, die nur grampositive Erreger enthalten. Gemäß ASTM E2871-13 oder ASTM E2871 - 21 kann hier eine Reduktion von CFU um mindestens $\log_{10} = 7$, bevorzugt um mindestens $\log_{10} = 8$ und ganz besonders bevorzugt um mindestens $\log_{10} = 9$ erreicht werden - insbesondere gegenüber *S. aureus.* Die Kontaktzeit zwischen erfindungsgemäßem Hydrogel bzw. Wundauflage und dem Biofilm kann hierbei 24h betragen. Eine Reduktion des Biofilms um $\log_{10} = 9$ in einem Test nach ASTM E2871-13 kann mit einer vollständigen Abtötung der im Biofilm befindlichen Organismen gleichgesetzt werden. Bevorzugt hat ein solches Hydrogel einen Wirkstoffgehalt von 0,5 bis 2,5 Gew.-%. Weitere Details hierzu können den Ausführungsbeispielen entnommen werden.

Im Rahmen der vorliegenden Erfindung ist der Komplex bzw. die Komplexverbindung $Ag_2Zn(EDTA)$ in das Hydrogel normalerweise eingebettet. Im Vergleich zu einer Beschichtung eines Hydrogels mit einem Wirkstoff führt dies zu einem stabileren Zusammenhalt und einer gleichmäßigeren Freisetzung des Wirkstoffs. Darüber hinaus bleiben die Mikroporen im Hydrogel frei und werden nicht versiegelt, was zu einem besseren Stoffaustausch (Freisetzung von Feuchtigkeit, Absorption von Exsudat und Erregern) führt. Gemäß einer bevorzugten Ausführungsform sind 95 Gew.-% des im Reaktionsgemisch eingesetzten Wirkstoffs in das Hydrogel eingebettet, besonders bevorzugt 98 Gew.-% und ganz besonders bevorzugt 99 Gew.-%.

Gemäß einer Ausführungsform enthält das erfindungsgemäße Hydrogel 0,5 bis 4 Gew.-% des Komplexes bzw. der Komplexverbindung $Ag_2Zn(EDTA)$, bevorzugt 0,5 bis 2,5 Gew.-% $Ag_2Zn(EDTA)$, besonders bevorzugt 0,5 bis 2 Gew.-% $Ag_2Zn(EDTA)$ und ganz besonders bevorzugt 1 bis 1,5 Gew.-% $Ag_2Zn(EDTA)$. Die Konzentration von $Ag_2Zn(EDTA)$ im Hydrogel kann über das erfindungsgemäße Herstellungsverfahren eingestellt werden.

Messungen haben gezeigt, dass der Wirkstoff in größerer Menge als dies zu erwarten gewesen wäre aus dem Hydrogel an die Wunde abgegeben wird. Es hat sich überraschenderweise herausgestellt, dass der Wirkstoff - neben seiner Wirksamkeit gegen Bakterien und Biofilme - weiterhin auch die Freisetzung von Feuchtigkeit aus der erfindungsgemäßen Hydrogelschicht verstärkt. Das heißt, dass ein Hydrogel, das gemäß dem erfindungsgemäßen Verfahren aber ohne Wirkstoff hergestellt wurde, weniger Feuchtigkeit an eine Wunde abgibt als ein erfindungsgemäßes wirkstoffhaltiges Hydrogel. Dieser Effekt wird darauf zurückgeführt, dass der Wirkstoff die Quervernetzung innerhalb der Hydrogelmatrix beeinflusst und damit auch die Neigung des Hydrogels Wasser bzw. Feuchtigkeit abzugeben.

Gemäß einer Ausführungsform beträgt die Feuchtigkeitsabgabe der erfindungsgemäßen Hydrogele mindestens 5 mg pro Quadratzentimeter und Tag. Mit zunehmendem Wirkstoffgehalt nimmt die Feuchtigkeitsabgabe zu. Bevorzugt beträgt die Feuchtigkeitsabgabe mindestens 8 mg pro $cm^2$ und Tag, besonders bevorzugt mindestens 15 mg pro $cm^2$ und Tag und ganz besonders bevorzugt mindestens 18 mg pro $cm^2$ und Tag. Hydrogele, die im Aufbau mit den erfindungsgemäßen Hydrogelen übereinstimmen, aber keinen Wirkstoff enthalten, zeigen eine Feuchtigkeitsabgabe von weniger als 5 mg pro $cm^2$ und Tag. Die Feuchtigkeitsabgabe kann hierbei in Bezug zu einem Filterpapier gesetzt werden, was bedeutet, dass die gemessene abgegebene Feuchtigkeit an das Filterpapier abgegeben wird. Bei dem Filterpapier kann es sich um ein handelsübliches Laborfilterpapier mit einem Durchmesser von 5 cm handeln. Das Laborfilterpapier kann einen durchschnittlichen Porendurchmesser von 15-20 $\mu$m und / oder eine durchschnittliche Filtrationsrate von 35 und 37 Sekunden aufweisen. Die Feuchtigkeitsabgabe geht mit einer Abgabe von Wirkstoff einher. Die Feuchtigkeitsabgabe kann mit der im vorliegenden Dokument beschriebenen Methode bestimmt werden (siehe Beispiel 1.4). In diesem Sinne umfasst die Erfindung die Verwendung eines Komplexes $Ag_2Zn(EDTA)$ in einem Hydrogel, um die Feuchtigkeitsabgabe des Hydrogels einzustellen oder zu steigern. Dabei gilt, dass ab einem Wirkstoffgehalt von 0,5 Gew.-% die Feuchtigkeitsabgabe mindestens 5 mg pro $cm^2$ und Tag beträgt, ab einem Wirkstoffgehalt von 1 Gew.-% mindestens 15 mg pro $cm^2$ und Tag und ab einem Wirkstoffgehalt von 1,5 Gew.-% mindestens 18 mg pro $cm^2$ und Tag.

Der auf diese Weise abgegebene Wirkstoff ist in der Lage eine mehrfache Wirkung zu entfalten. Er wirkt schädlichen Effekten entgegen, die durch Bakterien verursacht werden (Infektion, Biofilme, Entzündungen) und verbessert auf der anderen Seite die Wundheilung, indem er die Wunde mit Feuchtigkeit versorgt und durch einen Reinigungseffekt säubert. Durch den Reinigungseffekt können schädliche oder übermäßig vorhandene Stoffe aus dem Wundbett entfernt oder zumindest verdünnt und optional in eine absorbierenden Schicht der erfindungsgemäßen Wundauflage aufgenommen werden.

Erfindungsgemäße Hydrogele sind bevorzugt Bestandteil einer Wundauflage. Unter einer Wundauflage wird im Rahmen der vorliegenden Erfindung ein Produkt verstanden, das geeignet ist, auf eine Wunde aufgebracht zu werden und in gebrauchsfertiger Form zur Verfügung gestellt wird. Messung haben gezeigt, dass erfindungsgemäße Hydrogele mit einem Wassergehalt ab 60 Gew.-% eine besonders effektive Freisetzung des im Hydrogel als Wirkstoff enthaltenen Komplexes zeigen. Derartige Hydrogele und diese enthaltenen Wundauflagen stellen bevorzugte Ausführungsformen der Erfindung dar und sind in besonderem Maße dazu geeignet Biofilme in Wunden zu reduzieren oder vollständig aufzulösen.

Gemäß einer bevorzugten Ausführungsform umfassen erfindungsgemäße Wundauflagen mindestens das erfindungsgemäße Hydrogel als Wundkontaktschicht sowie eine der Wundkontaktschicht gegenüberliegende Trägerschicht, welche optional einen umlaufenden adhäsiven Bereich umfasst.

Die Wundkontaktschicht bietet verschiedene Vorteile. Hierzu gehört, dass bei einem Verbandswechsel eine besonders gewebeschonende Ablösung der erfindungsgemäßen Wundauflage gewährleistet wird. Weiterhin kann die Wundkontaktschicht die Wunde desinfizieren, bakterieller Besiedelung der Wunde vorbeugen oder eine solche reduzieren, die Wunde mit Feuchtigkeit versorgen, den Heilungsprozess beschleunigen, wundrandpflegende Eigenschaften aufweisen, Hautirritationen vermindern sowie antiadhärent wirken. Die erfindungsgemäßen Hydrogele eignen sich hervorragend als Wundkontaktschicht. Sie verkleben nicht mit der Wunde und verhindern ein Einwachsen von Granulationsgewebe in die Wundauflage.

Die Trägerschicht hat insbesondere die Funktion eine Austrocknung des Hydrogels an der wundabgewandten Seite zu verhindern. Eine geringe Abgabe von Feuchtigkeit bzw. Wasserdampf durch die Trägerschicht hindurch kann jedoch möglich bleiben. Erfindungsgemäße Wundauflagen können folgende Schichten umfassen: eine Trägerschicht, eine Hydrogelschicht gemäß der vorliegenden Erfindung und vorzugsweise eine zwischen der Hydrogelschicht und der Trägerschicht angeordnete absorbierende Schicht. Demnach umfasst die Wundauflage weiterhin zwischen der Wundkontaktschicht und der Trägerschicht vorteilhafterweise eine absorbierende Schicht, vorzugsweise in Form eines absorbierenden Schaumstoffs, besonders bevorzugt in Form eines absorbierenden Schaumstoffs aus Polyurethan. Beispielsweise kann eine wundzugewandte Seite des Polyurethanschaums mit dem Hydrogel beschichtet sein, während eine wundabgewandte Seite des Polyurethanschaums mit der Trägerschicht verbunden ist. Ferner kann bei dieser Ausführungsform das Hydrogel als netzförmige Wundkontaktschicht ausgestaltet sein. Auf diese Weise kann der Durchtritt von Wundexsudat in die zusätzliche absorbierende Schicht verbessert werden.

Der Wirkstoff verhindert, dass sich in die Wundauflage eingewanderte oder aufgenommene Bakterien in dieser vermehren können. Da aus der Wunde stammende Bakterien vor dem Erreichen einer absorbierenden Schicht die Hydrogelschicht (Wundkontaktschicht) durchqueren müssen, kommen sie zwangsläufig mit dem Wirkstoff in Kontakt. Auf diese Weise wird einer anschließenden Vermehrung der Keime in der absorbierenden Schicht vorgebeugt.

Als Trägerschicht können insbesondere Polymerfilme oder Polymerschäume eingesetzt werden, vorzugsweise Filme oder Schäume, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässiger und wasserdampfdurchlässiger Polyurethanfilm oder ein wasserundurchlässiger und wasserdampfdurchlässiger Polyurethanschaum geeignet. In besonders bevorzugten Ausführungsformen weisen diese Filme einen feuchtigkeitsdichten, umlaufenden adhäsiven Bereich auf. Dieser Bereich gewährleistet, dass das Wundsystem an seinem bestimmungsgemäßen Ort appliziert und fixiert werden kann. Darüber hinaus ist sichergestellt, dass keine Flüssigkeit zwischen der Folie und der an die Wunde angrenzende Haut austreten kann. Mögliche Klebstoffe sind Acrylklebstoffe bzw. Klebstoffe auf Acrylbasis. Diese gewährleisten eine besonders starke Haftung. Mögliche weitere Klebstoffe sind Silikonkleber bzw. Klebstoffe auf Silikonbasis. Diese ermöglichen eine atraumatische Haftung und sind besonders geeignet bei geschädigter oder empfindlicher Haut sowie häufigem Verbandwechsel. Der umlaufende adhäsive Bereich kann die in diesem Abschnitt aufgeführten Klebstoffe als Klebstoffschicht umfassen.

Mögliche Anordnungen der verschiedenen Schichten in erfindungsgemäßen mehrschichtigen Wundauflagen sind beispielsweise in der WO 2010/000450 beschrieben, auf welche hiermit vollumfänglich Bezug genommen wird.

Ferner kann die Wundauflage auch weitere Schichten neben der absorbierenden Schicht und der Trägerschicht umfassen, wie beispielsweise eine oder mehrere Barriereschichten und/oder eine oder mehrere Verteilerschichten.

Es ist üblich die Wundauflage mit einer Abdeck- bzw. Abziehfolie an der Wundkontaktseite bereitzustellen. Damit kann die Wundauflage während der Lagerung vor Kontamination geschützt und ein Feuchtigkeitsverlust vermieden werden. Die Abdeckfolie ist vor der Anwendung zu entfernen.

Die Wundauflage kann eine rechteckige oder im Wesentlichen quadratische Grundform aufweisen. Bevorzugt ist dabei ein Größenbereich von 8 cm x 8 cm bis zu 20 cm x 20 cm. Die Dicke der Wundauflage beträgt bevorzugt weniger als 2 cm, wobei eine mögliche Schaumschicht bevorzugt eine Dicke zwischen 0,1 cm und 1,8 cm, bevorzugt zwischen 0,3 cm und 0,8 cm, aufweist.

Als besonders vorteilhafte Ausführungen haben sich weiterhin Wundauflagen herausgestellt, die eine Hydrogelmatrix oder eine Wundkontaktschicht aus Hydrogel umfassen, deren Schichtdicke 0,1 bis 5,0 mm beträgt. Insbesondere weist damit eine erfindungsgemäße Wundauflage eine Wundkontaktschicht mit einer Schichtdicke von 0,1 bis 5,0 mm, insbesondere von 0,5 bis 5,0 mm und ganz besonders bevorzugt von 0,5 bis 3,0 mm auf. Wundauflagen mit solchen Schichtdicken zeigen die Fähigkeit ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen. Die erfindungsgemäßen Hydrogele sind für die Behandlung von Wunden geeignet. Die vorliegende Erfindung umfasst daher auch erfindungsgemäße Hydrogele zur Behandlung von Wunden. Insbesondere umfasst die vorliegende Erfindung Hydrogele zur Behandlung infizierter Wunden, chronischer Wunden wie Dekubitus, Druck-Ulzera, Druckgeschwüre, Ulcus cruris venosum, venöse Ulzera, Ulcus cruris arteriosum, arterielle Ulzera, Wunden infolge von diabetischem Fußsyndrom, neuropathische Ulzera, aber auch Wunden in Folge von Autoimmunerkrankungen oder von Tumoren (exulzierende Tumore) oder von Strahlenschäden bei der Tumortherapie.

In diesem Sinne umfasst die Erfindung auch ein Verfahren zur Behandlung von Wunden - insbesondere infizierten Wunden - und von Biofilmen in Wunden umfassend die folgenden Schritte:

1) Aufbringen des erfindungsgemäßen Hydrogels oder der erfindungsgemäßen Wundauflage auf eine Wunde bzw. auf eine infizierte oder mit Biofilm besiedelte Wunde,
2) Optionales Fixieren oder Abdecken des Hydrogels oder der erfindungsgemäßen Wundauflage, wobei das Fixieren oder Abdecken mithilfe eines umlaufenden adhäsiven Bereiches (im Falle einer Wundauflage), mithilfe von Klebestreifen, einer Binde und/oder einer Kompresse erfolgen kann,
und
3) Optionaler Wechsel des Hydrogels bzw. der Wundauflage nach 2 bis 7 Tagen, bevorzugt nach 3 bis 6 Tagen.

Erfindungsgemäße Hydrogele bzw. Wundauflagen, die diese enthalten, sind zur phasengerechten Wundtherapie geeignet, insbesondere zur Therapie von Wunden in der Granulationsphase und/oder der Epithelisierungsphase.

Beispiele

Die vorliegende Erfindung wird durch die nachfolgenden, nicht einschränkenden Beispiele im Detail dargestellt.

1. Hydrogele ohne Schaum

Beispiel 1.1 Herstellung der Hydrogele ohne Wirkstoff

Hydrogel-Prototypen wurden auf zwei verschiedene Arten hergestellt, nämlich manuell oder mit einer Gelgießanlage. Die Hauptprobenvorbereitung wurde mit der von bdtronic vertriebenen Gelanlage B100 durchgeführt, die eine dynamische Kontrolle und Kombination von zwei Komponenten unter Einstellung eines präzisen Mischungs-verhältnisses ermöglicht. Es können identische Prototypen mit gleicher Dicke und gleichem Gewicht/Flächenverhältnis hergestellt werden.

Zur Produktoptimierung wurden zusätzlich Hydrogele unter Laborbedingungen manuell hergestellt. Aufgrund der unterschiedlichen Reaktionsgeschwindigkeit bei verschiedenen Mischungsverhältnissen mussten einige Proben zügig zusammengeführt und direkt in Petrischalen verteilt werden, um ein Aushärten unter Rühren zu vermeiden. Im Gegensatz zur Gelmaschine war diese Methode eher für kleine Mengen von Hydrogelen mit unterschiedlichen Konzentrationen geeignet.

Die folgende Tabelle zeigt die prozentuale Verteilung der Bestandteile für die Hydrogelbildung:

Tabelle 1

| Lösung | Komponente | Anteil im Hydrogel [Gew.-%] | Lösungsanteil [Gew.-%] |
|---|---|---|---|
| A | Glycerin | 16,88 | 87 |
| | Jeffamin | 7,58 | |
| | Wasser | 62,53 | |
| B | Aquapol | 13 | 13 |

Die Gelanlage B100 besteht aus einem dynamischen Zweikomponenten-Mischkopf mit Materialzuführung über zwei pneumatisch gesteuerte Nadelventile, den beiden Vorratsbehältern für die Lösung von Alkohol-Amin-Gemisch und Aquapol sowie der Steuereinheit mit Digitalanzeige. Außerdem verfügt die Anlage über eine Druckluftaufbereitungseinheit. Die Lösungen A und B (s. Tabelle) wurden in die beiden Kartuschen eingefüllt. Bei diesem Füllvorgang besteht die Gefahr, dass sich Luft in den Lösungen einschließt. Damit der Gießprozess dadurch nicht gestört wird, wurden beide Lösungen zum Ausgasen zwölf Stunden ruhen gelassen. Die Komponenten wurden von externen Pumpen über separate Schläuche zu Nadelventilen gefördert, die am Mischkopf angebracht sind. Da die beiden Komponenten hier miteinander reagieren, muss die Mischzeit unterhalb der Gelbildungszeit gehalten werden, da es sonst zu Verhärtungen innerhalb des Mischkopfes kommen würde. Um einen konstanten Massenstrom, unabhängig von der Viskosität des Mediums, zu gewährleisten, wurden die Kartuschen mit Druckluft beaufschlagt. Der eingestellte Druck betrug beidseitig 1,5 bar. Um Feuchtigkeit im System zu vermeiden, wurde die Druckluft zuvor in einem Silikagel-Filter getrocknet. Das erzeugte Gel wurde über die Auslassdüse am Mischkopf ausgestoßen und in Kunststoff-Petrischalen gegeben. Die maximale Dosierleistung betrug 3,50 g/s.

Da es sich bei dem System um ein Zweikomponenten-Mischsystem handelt, wurde vor der Gelproduktion eine Batch-Lösung aus Jeffamin, Glycerin und Wasser (Lösung A) hergestellt. Da das Gewichtsverhältnis zwischen Jeffamine und Aquapol für die Gelbildung verantwortlich ist, ist eine genaue Einstellung des richtigen Mischungsverhältnisses unerlässlich. Durch empirische Analysen wurde festgestellt, dass bei wirkstofffreien Gelen ein Massenverhältnis von Masse Aquapol zu Masse Jeffamin = 1,7 optimal ist. Massenverhältnisse von 1,3 bis 1,8 lieferten ebenfalls befriedigende Ergebnisse.

Ferner wurde herausgefunden, dass bei Zugabe des Wirkstoffs zu den Hydrogelen das besagte Gewichtsverhältnis angepasst werden musste, um optimale Ergebnisse zu erhalten. Dies wird im nachfolgenden Abschnitt weiter ausgeführt.

Beispiel 1.2 Herstellung von Gelen mit unterschiedlichen Wirkstoffgehalten

Es wurden Hydrogele nach dem in Beispiel 1 beschriebenen Verfahren erzeugt, allerdings mit unterschiedlichem Wirkstoffgehalt. Zu diesem Zweck wurde eine 2,5 Gew.-%ige Stammlösung des Wirkstoffs in Wasser angefertigt, die den Gelen in unterschiedlichen Anteilen beigemengt wurde. Unerwarteterweise wurde festgestellt, dass der zugegebene Wirkstoff den Quervernetzungsgrad der resultierenden Hydrogele reduzierte. Weitere Analysen ergaben, dass mit steigender Wirkstoffkonzentration eine zunehmende Erhöhung des Anteils an Isocyanat-terminierten Präpolymer in Relation zum Amin-terminierten Präpolymer die Ergebnisse verbesserte. Durch die gesteigerte Beimengung des Isocyanat-terminierten Präpolymers konnten Hydrogele mit exzellenter Stabilität erhalten werden. Die Zusammensetzung der unterschiedlichen Hydrogele ist in der nachfolgenden Tabelle 2 angegeben, wobei zum Vergleich auch nochmals das wirkstofffreie Gel aus Tabelle 1 mit aufgeführt ist. Der Anteil in der Reaktionsmischung ist jeweils in Gewichtsprozent angegeben.

Tabelle 2

| Lösung | Komponente | Anteil in der Reaktionsmischung bei einem Gel ohne Wirkstoff | Anteil in der Reaktionsmischung bei einem Gel mit 0,5 Gew.-% Wirkstoff | Anteil in der Reaktionsmischung bei einem Gel mit 1 Gew.-% Wirkstoff | Anteil in der Reaktionsmischung bei einem Gel mit 1,5 Gew.-% Wirkstoff |
|---|---|---|---|---|---|
| A | Glycerin | 16,88 | 14,57 | 13,36 | 11,16 |
| | Jeffamine | 7,58 | 7,58 | 7,58 | 7,58 |
| | $H_2O$ | 62,53 | 42,54 | 22,49 | 0 |
| | wässrige Wirkstoff-Stammlsg. 2,5 % | 0 | 19,99 | 40,08 | 62,53 |
| B | Aquapol | 13 | 15,31 | 16,52 | 18,72 |
| Verhältnis Aquapol / Jeffamine | | 1,7 | 2,02 | 2,18 | 2,47 |

EP 4 521 925 B1

Zur Herstellung der Lösung A wurde die entsprechende Menge an Jeffamine im Wasserbad für 30 min auf 50°C erhitzt. Anschließend wurde die angegebene Menge Wasser zugegeben. Die Komponenten wurden mithilfe eines magnetischen Rührers vermischt und währenddessen die notwendige Menge an Wirkstoff-Stammlösung beigemengt. Lösungen A und B wurden in die B100-Anlage gegeben und die Gele durch Vermischung der beiden Lösungen mithilfe der Anlage hergestellt.

Die Hydrogele aus Tabelle 1 und 2 wurden für die nachfolgend beschriebenen Versuche verwendet.

Beispiel 1.3 Kinetik der Herstellung der Hydrogele

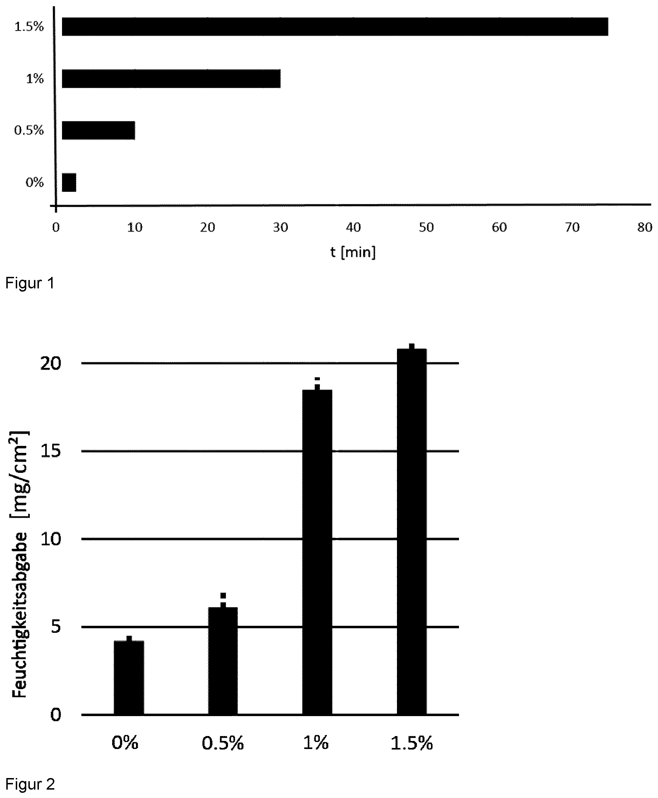

Es hat sich gezeigt, dass die Reaktionen zur Herstellung der Hydrogele unterschiedliche Reaktionsgeschwindigkeiten aufweisen. Die jeweiligen Reaktionsgeschwindigkeiten hingen vom Wirkstoffgehalt ab. Die Reaktion galt als abgeschlossen, sobald der sogenannte Gelierpunkt erreicht war und sich eine nicht klebrige gelartige Oberfläche ausbildete. Die Ergebnisse sind Fig. 1 dargestellt.

Während ein Referenzgel ohne Wirkstoff (Zusammensetzung gemäß Tab. 1) etwa 2 min benötigte, um den Gelierpunkt zu erreichen, waren es bei einem Wirkstoffgehalt von 0,5 % etwa 10 min, bei 1 % etwa 30 min und bei 1,5 % etwa 75 min.

Beispiel 1.4 Feuchtigkeitsabgabe der Hydrogele

Die Feuchtigkeitsabgabe an Filterpapier für erfindungsgemäße Hydrogele mit unterschiedlichen Wirkstoffgehalten wurde gemessen. Ein Gel ohne Wirkstoff (Zusammensetzung gemäß Tab. 1) wurde als Referenz in die Messung aufgenommen.

Für die Messung wurden Proben mit einem Durchmesser von 2,5 cm ausgestanzt und deren Ausgangsgewicht bestimmt. Anschließend wurden die Proben in mit Filterpapier ausgelegten Petrischalen platziert. Petrischalen und Filterpapier hatten einen Durchmesser von 5 cm und wurden ebenfalls zuvor gewogen. Die Petrischalen wurden versiegelt und bei einer Temperatur von 37°C inkubiert. Nach 24 h wurden die Proben sowie Filterpapier + Petrischale unabhängig voneinander gewogen. Die Feuchtigkeitsabgabe wurde nach der folgenden Formel bestimmt:

$$\text{Feuchtigkeitsabgabe} = \frac{(mt - m0) \times 1000}{A}$$

mt = Gewicht von Petrischale und Filterpapier nach 24 h [g]
m0 = Ausgangsgewicht von Petrischale und Filterpapier [g]
A = Fläche des Filterpapiers [cm$^2$]

Die Ergebnisse sind in der nachfolgenden Tabelle und zusätzlich als Balkendiagramm in Fig. 2 dargestellt:

Tabelle 3

| Wirkstoffkonzentration [%] | Feuchtigkeitsabgabe [mg/cm$^2$] |
|---|---|
| 0 | < 5 |
| 0,5 | 6,1 |
| 1 | 18,5 |
| 1,5 | 20,8 |

Beispiel 1.5 Wirkstofffreisetzung aus den Hydrogelen

Freisetzung von Silber und Zink

Gelproben mit einer Wirkstoffkonzentration von 1,5 % wurden zur Bestimmung der Freisetzung getestet. Für die Analyse von Spurenelementen im Konzentrationsbereich mg/L bis μg/L wurde optische Emissionsspektrometrie mit induktiv gekoppeltem Plasma verwendet (ICP-OES).

Diese Methode erlaubt die gleichzeitige Bestimmung aller Metalle und einige Nichtmetalle aus angesäuerten wässrigen Lösungen bis hin zu einem Gehalt von etwa 10 g/L.

Für die Bestimmung der Ionenfreisetzung von Silber und Zink wurden Hydrogelproben mit einem Gewicht von einem Gramm in ein Probengefäß überführt und 10 mL demineralisiertes Wasser zugegeben. Es wurden fünfzehn Proben angefertigt und bei 180 rpm bei Raumtemperatur auf einem Schüttler inkubiert. Nach 4, 24, 48, 72 Stunden und 7 Tagen wurden pro Probe jeweils 3 mL der Lösung in Fläschchen pipettiert. Diese Proben wurden nach EN ISO 17294-2 (E29) analysiert. Dabei wurden die Proben einem Druckaufschluss mit Salpetersäure in Teflongefäßen unterzogen.

Die resultierende klare Lösung wurde auf eine Endkonzentration von 10 - 50 g/L verdünnt und analysiert. Bei der ICP-OES-Analyse wurde die Probenlösung über ein pneumatisches Zerstäubersystem in ein induktiv gekoppeltes Argonplasma eingeführt. Bei einer Temperatur von 5000 - 7000 K im Plasma wurden die Elemente atomisiert und zur Lichtemission angeregt. Das emittierte Licht wurde bei der gleichzeitigen Analyse durch einen Polychromator erfasst, der in elementspezifische Wellenlängen aufgeteilt war. Um den Elementgehalt einer Lösung quantitativ zu bestimmen, wurde das Gerät mit synthetischen Lösungen mit bekanntem Gehalt kalibriert. Die Analyse der Migration von Zinkionen in 1,5 % igen Hydrogelen in $H_2O$ zeigte einen fast linearen Anstieg von 0,07 g/L nach 24 Stunden auf 0,13 g/L nach 7 Tagen. Es kann daher von einer konstanten Freisetzung der Ionen ausgegangen werden.

Im Gegensatz dazu zeigte die Analyse der freigesetzten Silberionen in demselben Medium nach etwa einem Tag einen starken Abfall der Freisetzung, bis nach etwa 72 Stunden ein gleichbleibend niedriges Niveau erreicht wurde. Während die Werte über einen Zeitraum von 24 Stunden konstant bei 0,19 g/L lagen, sank die nachweisbare Menge an Silberionen nach 7 Tagen auf 0,02 g/L.

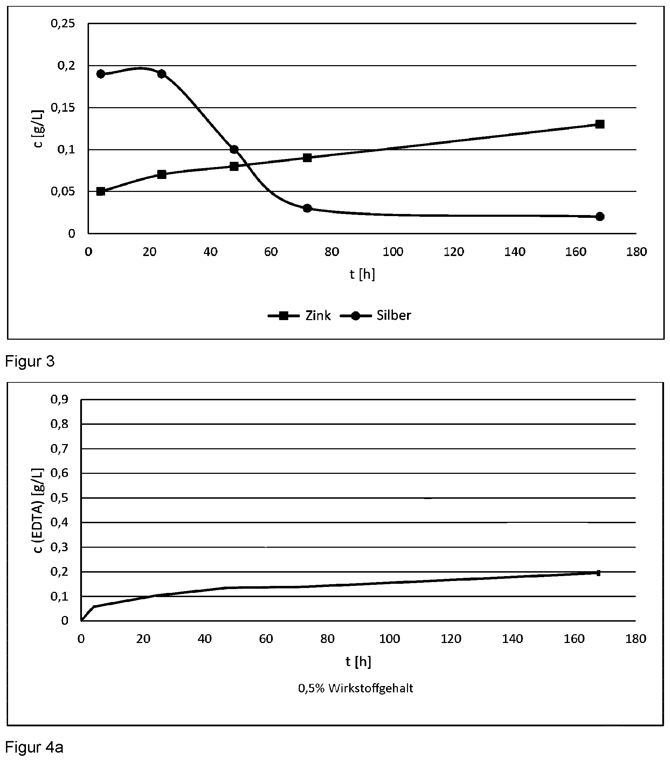

Die Ergebnisse sind in Fig. 3 dargestellt.

Freisetzung von EDTA

Die Freisetzung von EDTA wurde mittels UV/VIS-Spektroskopie bestimmt. Um die Migration des im Hydrogel enthaltenen EDTA in eine wässrige Lösung zu untersuchen, wurde das Eluat mit einem photoLab® S6 Photometer analysiert. Dazu wurde das Absorptionsmaximum von Natrium-EDTA in einer wässrigen Lösung geschätzt. Für die Entwicklung einer Kalibrierungskurve wurde eine serielle Verdünnung von NaEDTA-Konzentrationen im Bereich der berechneten maximalen Expositionsgrenzen von Hydrogelen hergestellt. Diese deckte den Konzentrationsbereich von 0 bis 1 g / L ab.

Um zunächst das Absorptionsmaximum des in den Hydrogelen vorhandenen EDTA-Komplexes zu ermitteln, wurde zunächst eine UV/VIS-Messung einer 1-molaren Natrium-EDTA-Komplex-Lösung (NaEDTA) durchgeführt. Dies ergab ein Maximum bei einer Wellenlänge von ṽ = 605 nm.

Für die Bestimmung der Freisetzung wurden Hydrogelproben mit einem Gewicht von 1 g in Probengefäße überführt und jeweils 10 ml demineralisiertes Wasser hinzugefügt. Die Proben wurden bei 180 U/min bei Raumtemperatur auf einem Schüttler inkubiert. Nach 4, 24, 48, 72 Stunden und 7 Tagen wurden 3 mL der Eluate in Polystyrolküvetten pipettiert und die Absorption in % bei einer Wellenlänge von 605 nm gemessen.

Die Absorptionsmessungen der erfindungsgemäßen Hydrogele bei einer Wellenlänge von 605 nm zeigten einen Trend zu einer zunehmenden Absorption proportional zur steigenden Wirkstoffkonzentration. Während die Hydrogele mit einer Konzentration von 0,5 % nach 24 Stunden eine Absorption von 0,034 % aufwiesen, stieg dieser Wert innerhalb von 7 Tagen auf fast das Doppelte (0,064 %) an. Eine Verdoppelung der Absorption wurde auch bei Hydrogelen mit 1 % Wirkstoff beobachtet, und zwar von 0,077 % nach 24 Stunden auf 0,134 % nach 7 Tagen. 1,5 %ige Hydrogele zeigten eine Absorption von 0,072 % nach 24 Stunden und 0,249 % nach 7 Tagen.

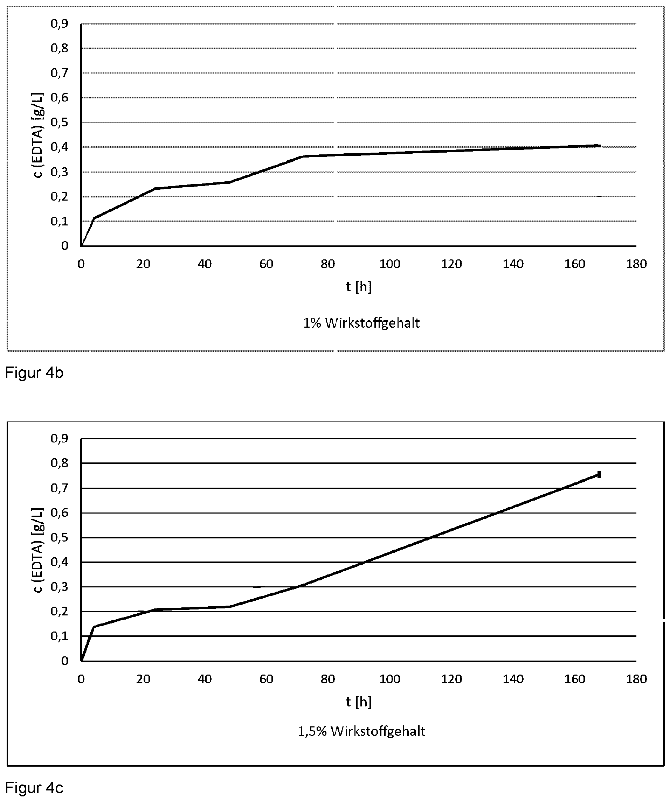

Unter Anwendung der zuvor erstellten Kalibrierungskurve (Absorption in Abhängigkeit von EDTA-Konzentration) betrug die maximale ins Medium übergegangene EDTA-Konzentration nach 7 Tagen 0,194 g/L für 0,5 %ige Hydrogele, 0,406 g/L für 1 %ige Hydrogele und 0,754 g/L für 1,5 %ige Hydrogele. Die Messwerte sind als Kurvendiagramme in den Fig. 4a (0,5 % Wirkstoffgehalt), 4b (1 % Wirkstoffgehalt) und 4c (1,5 % Wirkstoffgehalt) dargestellt.

Wie aus den Figuren ersichtlich wird, war die Konzentration der freigesetzten EDTA-Komplexe proportional zum Wirkstoffgehalt in den Hydrogelen.

Beispiel 1.6 Antimikrobielle Wirkung der Hydrogele gegenüber Biofilmen

Um die antimikrobielle Wirkung erfindungsgemäßer Hydrogele zu bestimmen, wurde die Wirksamkeit gegen Biofilme der beiden humanpathogenen bakteriellen Keime *S. aureus* (Stamm gemäß Hinterlegung ATCC 6538) und *P. aeruginosa* (Stamm gemäß Hinterlegung ATCC 15442) analysiert. Hierbei repräsentierte *S. aureus* die grampositiven Bakterien und *P. aeruginosa* die gramnegativen Bakterien. Der Test erfolgte gemäß dem öffentlich zugänglichen Standardprotokoll ASTM E2871 - 13. Zur Erzeugung der Biofilme wurden CDC-Bioreaktoren (Center for Disease Control) verwendet. Im Deckel dieser Bioreaktoren sind mehrere Stäbe eingelassen, welche bei Benutzung in die Nährlösung eintauchen. Die Stäbe haben Aussparungen, welche spezielle Plättchen - sogenannte Coupons - aufnehmen können. Während der Inkubation des Bioreaktors bilden sich auf den Coupons Biofilme aus. An diesen Biofilmen wurde die gegen Biofilme gerichtete Wirkung der erfindungsgemäßen Hydrogele getestet.

Zunächst wurde eine einzelne Kolonie jeder Art in 10 mL TBS (Tris-gepufferte Kochsalzlösung) inokuliert und über Nacht bei 37°C und 125 rpm auf einer Rüttelplatte inkubiert.

Die Übernachtkultur von *P. aeruginosa* wurde auf 108 CFU (koloniebildende Einheiten) pro ml eingestellt. Anschließend wurde sie zur Beimpfung eines CDC-Bioreaktors verwendet, indem 1 ml zu 300 ml TSB hinzugefügt wurde. Der Bioreaktor wurde in der Batchphase bei 37°C und 80 rpm auf einer Rüttelplatte für 24h inkubiert.

Die Übernachtkultur von *S. aureus* wurde für drei Minuten zentrifugiert, der Überstand verworfen und das Zellpellet in 1 ml TSB resuspendiert. Das resuspendierte Pellet wurde verwendet, um den CDC-Bioreaktor zu inokulieren, indem es zu 300 mL TSB gegeben wurde. Der Bioreaktor wurde in der Batch-Phase bei 37°C und 80 rpm auf einer Rüttelplatte für 24 h inkubiert.

Nach Ablauf der 24 h hatten sich Biofilme ausgebildet. Die Stäbe wurden aus dem Bioreaktor gezogen und zwei Mal mit PBS gewaschen. Die Coupons wurden in Hydrogele eingewickelt, die zuvor auf eine Größe von 2,5 x 5 cm zugeschnitten worden waren. Pro Ansatz wurden drei Coupons verwendet. Die eingewickelten Coupons wurden in 12-Well-Platten platziert und für 24 h bei Raumtemperatur (*P. aeruginosa*) oder 37°C (*S. aureus*) inkubiert.

Am darauffolgenden Tag wurden die Coupons aus den Wells entnommen, zu 10 mL Dey Engley neutralising broth gegeben und für 30 min mit Ultraschall behandelt.

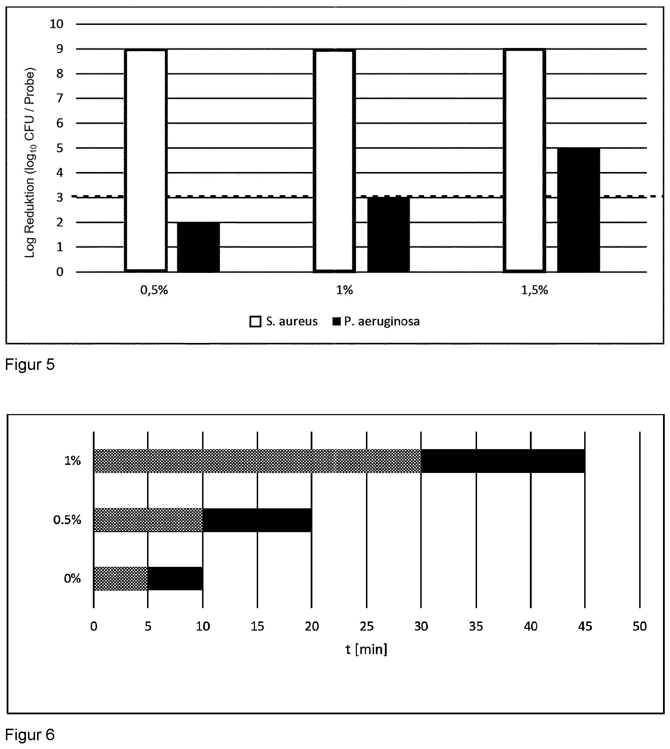

Anschließend wurden die Proben auf einem Vortex-Gerät geschüttelt und auf 96-Well-Platten verteilt. Jede Probe wurde im Verhältnis 1:10 seriell mit PBS verdünnt und 20 $\mu$l jeder Verdünnung wurden in jeweils zweifacher Ausführung auf TSA-Platten (Trypton-Soja-Agar) verteilt. Die Proben wurden über Nacht bei 37°C inkubiert und am nächsten Tag die Koloniezahlen ausgezählt. Hierdurch wurde die Gesamtzahl der lebensfähigen Keime bestimmt. Die Ergebnisse für Hydrogele mit einem Wirkstoffgehalt von 0,5 %, 1 % und 1,5 % sind als Diagramm in Fig. 5 dargestellt.

Gegenüber Biofilmen von *S. aureus* zeigten Hydrogele mit allen drei getesteten Wirkstoffkonzentrationen eine Reduktion der Kolonie bildenden Einheiten (CFU) von $\log_{10} = 9$ und damit eine extrem hohe Wirksamkeit. Da eine höhere Wirkstoffkonzentration keine weitere Reduktion der koloniebildenden Einheiten zur Folge hatte, kann davon ausgegangen werden, dass bereits die niedrigste Wirkstoffkonzentration von 0,5 % eine vollständige Abtötung sämtlicher im Biofilm befindlicher Bakterien zur Folge hatte.

Gegenüber Biofilmen von *P. aeruginosa* nahm der antimikrobielle Effekt mit steigender Wirkstoffkonzentration zu. Hydrogele mit einem Wirkstoffgehalt von 0,5 % zeigten eine logarithmische Reduktion von $\log_{10} = 2$, bei einem Wirkstoffgehalt von 1 % eine Reduktion von $\log_{10} = 3$ und bei 1,5 % von $\log_{10} = 5$. Somit kann davon ausgegangen werden, dass die Wirkung der erfindungsgemäßen Hydrogele gegenüber *P. aeruginosa* (und möglicherweise gegenüber gramnegativen Bakterien im Allgemeinen) dosisabhängig ist und mit steigender Wirkstoffkonzentration weiter zunimmt.

2. Hydrogele auf Polyurethanschaum

Beispiel 2.1 Herstellung wirkstoffhaltiger Hydrogele auf PU-Schaum

Es wurden Hydrogele mit einem Wirkstoffgehalt von 0,5 und 1 Gew.-% während ihrer Herstellung mit einem Polyurethanschaum verbunden. Als zusätzliche Referenz diente hierbei ein Hydrogel ohne Wirkstoff. Bei den durchgeführten Versuchen hat sich gezeigt, dass der Zeitpunkt der Aufbringung des Hydrogels auf den PU-Schaum entscheidend ist. Wenn die Hydrogele mit dem PU-Schaum in Kontakt gebracht werden, muss die Hydrogelmatrix noch ausreichend Vernetzungspotential haben. Auf der anderen Seite darf das Reaktionsgemisch nicht zu früh auf den Schaum aufgebracht werden, da es ansonsten vom Schaum absorbiert wird. Obwohl der optimale Zeitpunkt beim Referenzgel nach dem Gelierzeitpunkt lag, hat sich gezeigt, dass für die wirkstoffhaltigen Gele der Gelierzeitpunkt der optimale Zeitpunkt ist. Die Ergebnisse sind in Fig. 6 dargestellt: Der schraffierte Bereich der Diagrammbalken zeigt die Zeitdauer ab Reaktionsbeginn bis zu dem Zeitpunkt, an dem das Gel mit dem PU-Schaum in Kontakt gebracht wurde. Der schwarze Bereich der Diagrammbalken zeigt die Zeitdauer, in welcher die Gele sich mit dem PU-Schaum verbunden haben. Zu diesem Zweck wurden Schäume entsprechender Größe zu dem Gel in die Gussform gegeben. Der Vorgang des Verbindens wurde als abgeschlossen betrachtet, sobald der Gel-Schaum-Verbund aus der Gussform entnommen werden konnte, ohne sich zu trennen oder Schaden zu nehmen. Bei einem Wirkstoffgehalt von 0,5 Gew.-% wurden 10 min und bei einem Wirkstoffgehalt von 1 Gew.-% wurden 15 min benötigt, bis sich Gel und Schaum miteinander verbunden hatten. Das Ergebnis waren PU-Schäume, die mit erfindungsgemäßen Hydrogelen stabil beschichtet waren und aus der Gussform entnommen werden konnten.

Beispiel 2.2 Absorptionskapazität von Hydrogelen auf PU-Schaum

Zur Bestimmung der Absorptionskapazität des beschichteten PU-Schaums wurden Proben einer Größe von 2,5 x 2,5 cm aus den hydrogelbeschichteten Schäumen herausgestanzt. Jede Probe wurde gewogen und in einen Glasbecher mit demineralisiertem Wasser gestellt. Nach jeweils 24 Stunden wurden die Proben entnommen, mithilfe von Zellstoffpapier getrocknet und gewogen. Um die Absorptionskapazität für jede Probe zu bestimmen, wurde die folgende Formel verwendet:

Absorptionskapazität = (Endgewicht nach 24h - Ausgangsgewicht) / Ausgangsgewicht

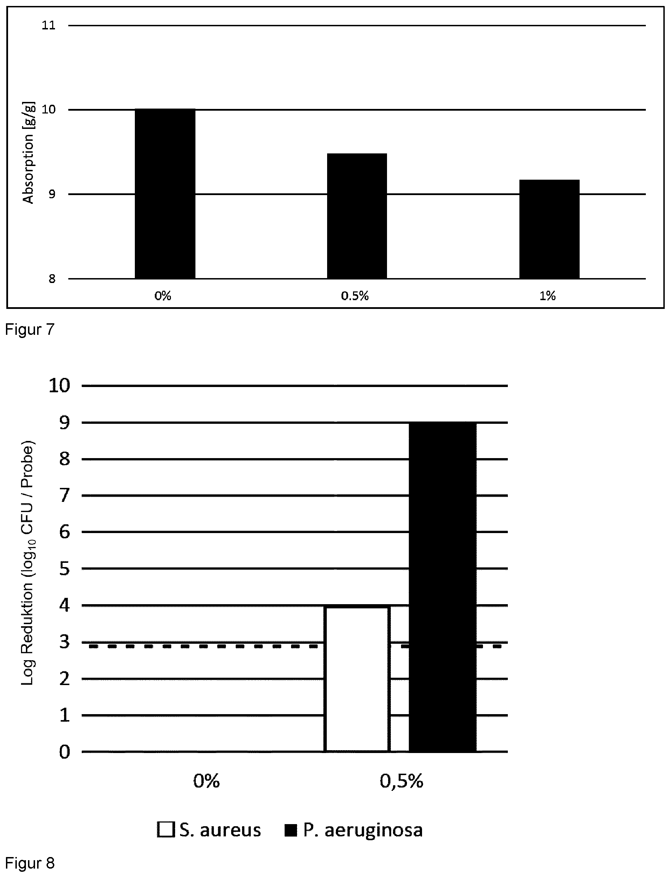

Nach 24 Stunden zeigten Hydrogele mit einem Wirkstoffgehalt von 0,5 % auf PU-Schaum eine Absorption von 9,47 g/g, wohingegen bei einem Wirkstoffgehalt von 1 % eine Absorption von 9,17 g/g gemessen wurde. Bei einem Referenzgel auf PU-Schaum ohne Wirkstoff wurden 10 g/g gemessen. Ein höherer Wirkstoffgehalt schein daher mit einer geringeren Absorption einherzugehen. Allerdings konnte durch andere Messungen gezeigt werden, dass ein höherer Wirkstoffgehalt auch gleichzeitig die Freisetzung von Feuchtigkeit aus dem Gel fördert, so dass vermutet werden kann, dass die höhere Feuchtigkeitsabgabe mit einer geringeren Absorptionsbereitschaft einhergeht. Die Ergebnisse sind in Fig. 7 als Diagramm dargestellt.

Beispiel 2.3 Adhäsionskraft von Hydrogelen auf PU-Schaum

Die Adhäsionskraft der mit PU-Schaum verbundenen Hydrogele wurde auf Stahl getestet. Zum Einsatz kam eine Zugprüfmaschine, welche dem Standard DIN EN ISO 7500-01 entsprach. Sämtliche Messungen fanden unter standardisierten Bedingungen von 23°C und 50 % relativer Luftfeuchtigkeit statt. Es wurden Proben einer Größe von 5 x 5 cm ausgestanzt. Die Proben wurden mit der Rückseite (Schaumseite) an einem horizontal beweglichen Träger mittels doppelseitigem Klebeband befestigt. Zur Durchführung der Messung wurde ein Metallgewicht mit einer Gewichtskraft von 0,245 N mit einer Unterseite aus Glas verwendet. Die Unterseite wurde vor Beginn der Messung mit einem Ethanol getränkten Pad gereinigt. Das Gewicht wurde mit der Unterseite voran auf dem Hydrogel platziert und die Messung gemäß der folgenden Parameter durchgeführt:

Tabelle 4

| Anfangsgeschwindigkeit [mm / min] | Abziehgeschwindigkeit [mm / min] | Kontaktzeit [s] |
| --- | --- | --- |
| 100 | 400 | 2 |

Nach Ablauf der Kontaktzeit wurde mittels der Zugprüfmaschine die zum Abziehen des Gewichts in einem 90°-Winkel notwendige Kraft gemessen. Es wurden pro Wirkstoffgehalt jeweils fünf Messungen durchgeführt und dabei die folgenden Durchschnittswerte ermittelt:

Tabelle 5

| Wirkstoffgehalt [%] | Adhäsionskraft [N] |
| --- | --- |
| 0 | 0.767 |
| 0,5 | 0.834 |
| 1 | 0.867 |

Wie zu erkennen ist, stieg die Adhäsionskraft mit zunehmender Wirkstoffkonzentration überraschenderweise an.

Beispiel 2.4 Antimikrobielle Wirkung der Hydrogele auf PU-Schaum gegenüber Biofilmen

Der weiter oben beschriebene Test auf antibakterielle Wirksamkeit der Hydrogele gegenüber Biofilmen wurde wiederholt. Diesmal wurden Hydrogele mit einer Wirkstoffkonzentration von 0 % und 0,5 %, die als Beschichtung auf PU-Schaum angebracht waren, getestet. Die Ergebnisse sind in Fig. 8 dargestellt.
Wie aus den Ergebnissen ersichtlich, zeigen Hydrogele auf PU-Schaum ohne Wirkstoff keine Wirkung gegenüber Biofilmen von *S. aureus* oder *P. aeruginosa*. Hingegen ist bei einer Wirkstoffkonzentration von 0,5 % eine antibakterielle Wirkung gegenüber den Biofilmen messbar. So wurde der Biofilm von *S. aureus* um einen Logarithmus von $\log_{10} = 4$ reduziert. Beim Biofilm von *P. aeruginosa* fand sogar eine Reduktion von $\log_{10} = 9$ statt. Wie in den zuvor präsentierten Ergebnissen der antibakteriellen Wirkung von Hydrogelen ohne Schaum diskutiert, kann bei einer Reduktion von $\log_{10} = 9$ davon ausgegangen werden, dass sämtliche Bakterien innerhalb des Biofilms abgetötet wurden.
Ausgehend von den hier vorgestellten Ergebnissen kann vermutet werden, dass erfindungsgemäße Hydrogele als Beschichtung von PU-Schaum eine besonders hohe Wirksamkeit gegenüber Biofilmen gramnegativer Bakterien wie *P. aeruginosa* entfalten und erfindungsgemäße Hydrogele ohne Schaum eine besonders hohe Wirksamkeit gegenüber Biofilmen grampositiver Bakterien wie *S. aureus* erzielen.

Bevorzugt umfasst die Erfindung des Weiteren:

Erfindungsgemäßes Hydrogel , wobei die Feuchtigkeitsabgabe des Hydrogels in einem Zeitraum von 24 h mindestens 5 mg pro Quadratzentimeter, bevorzugt 10 mg pro Quadratzentimeter, besonders bevorzugt 15 mg pro Quadratzentimeter und ganz besonders bevorzugt 20 mg pro Quadratzentimeter beträgt.

Die Verwendung eines Komplexes $Ag_2Zn(EDTA)$ in einem Hydrogel, um die Adhäsionskraft des Hydrogels einzustellen oder zu steigern.

Die Verwendung eines Komplexes $Ag_2Zn(EDTA)$ in einem Hydrogel, um die Feuchtigkeitsabgabe des Hydrogels einzustellen oder zu steigern.

## Patentansprüche

1. Verfahren zur Herstellung eines Biofilm reduzierenden Hydrogels für medizinische Zwecke, insbesondere für die Wundbehandlung, umfassend die folgenden Schritte:

   i. Bereitstellen eines Isocyanat-terminierten Präpolymers enthaltend Polyalkylenoxideinheiten,
   ii. Bereitstellen eines Amin-terminierten Präpolymers enthaltend Polyalkylenoxideinheiten,
   iii. Lösen der unter ii bereitgestellten Substanz in einer Wasser enthaltenden Flüssigkeit, um eine wässrige Zubereitung zu erhalten,
   iv. Mischen der wässrigen Zubereitung und einer Lösung, insbesondere einer wässrigen Lösung, enthaltend einen Komplex $Ag_2Zn(EDTA)$, um eine Ausgangslösung zu erhalten,
   v. Zusammenführen der Ausgangslösung und des Isocyanat-terminierten Präpolymers zu einer Reaktionsmischung, wodurch diese durch Polymerisation zum Hydrogel umgesetzt werden,

   wobei der pH-Wert der Lösung aus Schritt iv mindestens 9, bevorzugt 10 bis 12, ist und wobei die Reaktionsmischung aus Schritt v keine Acrylsäure oder Polyacrylsäure enthält.

2. Verfahren nach Anspruch 1, **dadurch gekennzeichnet, dass** das Massenverhältnis des Isocyanat-terminierten Präpolymers zum Amin-terminierten Präpolymer zwischen 1,3 und 3,2 liegt.

3. Verfahren nach Anspruch 1 oder 2, wobei die Wasser enthaltende Flüssigkeit in Schritt iii Glycerin enthält.

4. Verfahren nach einem der vorhergehenden Ansprüche, wobei die Reaktionsmischung 10 bis 30 Gew.-% Glycerin enthält.

5. Verfahren nach einem der vorhergehenden Ansprüche, wobei der pH-Wert der Lösung aus Schritt iv mittels Ammoniak, Natriumhydroxid oder Essigsäure eingestellt wird.

6. Verfahren nach einem der vorhergehenden Ansprüche, wobei die Reaktionsmischung kein Triethanolamin, keine Chloridionen und / oder Chlorsalze, insbesondere kein NaCl, enthält.

7. Verfahren nach einem der vorhergehenden Ansprüche, wobei die Reaktionsmischung aus Schritt v 0,5 bis 4 Gew.-% des Komplexes $Ag_2Zn(EDTA)$ enthält.

8. Verfahren nach einem der vorhergehenden Ansprüche, wobei die Summe der Massen aus Amin-terminiertem Präpolymer und Isocyanat-terminiertem Präpolymer 10 bis 30 Gew.-% der Reaktionsmischung beträgt.

9. Hydrogel mit Biofilm reduzierenden Eigenschaften erhältlich durch das Verfahren nach einem der vorhergehenden Ansprüche.

10. Hydrogel nach Anspruch 9, wobei das Hydrogel einen Wassergehalt von 40 bis 65 Gew.-% hat.

11. Hydrogel nach Anspruch 9 oder 10, wobei das Hydrogel einen pH-Wert von 6,5 bis 9,5, vorzugsweise 6,5 bis 8, hat.

12. Hydrogel nach einem der Ansprüche 9 bis 11, wobei das Hydrogel die Anzahl bakterieller Zellen von *S. aureus* oder *P. aeruginosa* innerhalb eines Biofilms während eines Tests gemäß ASTM E2871-13 und einer Kontaktzeit von 24 h um mindestens $\log_{10} = 2$ reduziert.

13. Hydrogel nach einem der Ansprüche 9 bis 12, wobei das Hydrogel 0,5 bis 4 Gew.-% des Komplexes $Ag_2Zn(EDTA)$ enthält.

14. Hydrogel nach einem der Ansprüche 9 bis 13, wobei die Feuchtigkeitsabgabe des Hydrogels mindestens 5 mg pro Quadratzentimeter und Tag beträgt.

15. Wundauflage umfassend ein Hydrogel nach einem der Ansprüche 9 bis 14 als Wundkontaktschicht, sowie eine der Wundkontaktschicht gegenüberliegende Trägerschicht, wobei die Trägerschicht optional einen umlaufenden adhäsiven Bereich umfasst.

16. Wundauflage nach Anspruch 15, weiterhin umfassend zwischen der Wundkontaktschicht und der Trägerschicht einen absorbierenden Schaumstoff, vorzugsweise einen absorbierenden Schaumstoff aus Polyurethan.

**Claims**

1. Process for producing a biofilm-reducing hydrogel for medical purposes, especially for wound treatment, comprising the following steps:

    i. providing an isocyanate-terminated prepolymer containing polyalkylene oxide units,
    ii. providing an amine-terminated prepolymer containing polyalkylene oxide units,
    iii. dissolving the substance provided in ii in a water-containing liquid in order to obtain an aqueous formulation,
    iv. mixing the aqueous formulation and a solution, especially an aqueous solution, containing an $Ag_2Zn(EDTA)$ complex, in order to obtain a starting solution,
    v. combining the starting solution and the isocyanate-terminated prepolymer to give a reaction mixture, whereby these are converted by polymerization to the hydrogel,

    wherein the pH of the solution from step iv is at least 9, preferably 10 to 12, and wherein the reaction mixture from step v does not contain any acrylic acid or polyacrylic acid.

2. Process according to Claim 1, **characterized in that** the mass ratio of the isocyanate-terminated prepolymer to the amine-terminated prepolymer is between 1.3 and 3.2.

3. Process according to Claim 1 or 2, wherein the water-containing liquid in step iii contains glycerol.

4. Process according to any of the preceding claims, wherein the reaction mixture contains 10% to 30% by weight of glycerol.

5. Process according to any of the preceding claims, wherein the pH of the solution from step iv is adjusted by means of ammonia, sodium hydroxide or acetic acid.

6. Process according to any of the preceding claims, wherein the reaction mixture does not contain any triethanolamine, any chloride ions and/or chlorine salts, in particular any NaCl.

7. Process according to any of the preceding claims, wherein the reaction mixture from step v contains 0.5% to 4% by weight of the $Ag_2Zn(EDTA)$ complex.

8. Process according to any of the preceding claims, wherein the sum total of the masses of amine-terminated prepolymer and isocyanate-terminated prepolymer is 10% to 30% by weight of the reaction mixture.

9. Hydrogel having biofilm-reducing properties, obtainable by the process according to any of the preceding claims.

10. Hydrogel according to Claim 9, wherein the hydrogel has a water content of 40% to 65% by weight.

11. Hydrogel according to Claim 9 or 10, wherein the hydrogel has a pH of 6.5 to 9.5, preferably 6.5 to 8.

12. Hydrogel according to any of Claims 9 to 11, wherein the hydrogel reduces the number of bacterial cells of *S. aureus* or *P. aeruginosa* within a biofilm during a test according to ASTM E2871-13 and over a contact time of 24 h by at least

$\log_{10} = 2$.

13. Hydrogel according to any of Claims 9 to 12, wherein the hydrogel contains 0.5% to 4% by weight of the $Ag_2Zn$ (EDTA) complex.

14. Hydrogel according to any of Claims 9 to 13, wherein the release of moisture from the hydrogel is at least 5 mg per square centimetre and day.

15. Wound dressing comprising a hydrogel according to any of Claims 9 to 14 as wound contact layer, and a carrier layer opposite the wound contact layer, wherein the carrier layer optionally comprises a circumferential adhesive region.

16. Wound dressing according to Claim 15, further comprising an absorbing foam between the wound contact layer and the carrier layer, preferably an absorbing polyurethane foam.


**Revendications**

1. Procédé de préparation d'un hydrogel, réduisant les biofilms, destiné à des fins médicales, en particulier pour le traitement de plaies, comprenant les étapes suivantes :

   i. mise à disposition d'un prépolymère terminé par isocyanate contenant des motifs poly(oxyde d'alkylène),
   ii. mise à disposition d'un prépolymère terminé par amine contenant des motifs poly(oxyde d'alkylène),
   iii. dissolution de la substance mise à disposition dans l'étape ii dans un liquide contenant de l'eau, afin d'obtenir une préparation aqueuse,
   iv. mélange de la préparation aqueuse et d'une solution, en particulier d'une solution aqueuse, contenant un complexe $Ag_2Zn(EDTA)$, afin d'obtenir une solution de départ,
   v. réunion de la solution de départ et du prépolymère terminé par isocyanate en un mélange réactionnel, suite à quoi ceux-ci sont transformés en hydrogel par polymérisation,

   la valeur du pH de la solution de l'étape iv étant d'au moins 9, de préférence de 10 à 12, et le mélange réactionnel de l'étape v ne contenant pas d'acide acrylique ni de poly(acide acrylique).

2. Procédé selon la revendication 1, **caractérisé en ce que** le rapport massique du prépolymère terminé par isocyanate au prépolymère terminé par amine se situe entre 1,3 et 3,2.

3. Procédé selon la revendication 1 ou 2, le liquide contenant de l'eau dans l'étape iii contenant du glycérol.

4. Procédé selon l'une des revendications précédentes, le mélange réactionnel contenant 10 à 30 % en poids de glycérol.

5. Procédé selon l'une des revendications précédentes, la valeur du pH de la solution de l'étape iv étant réglée au moyen d'ammoniaque, d'hydroxyde de sodium ou d'acide acétique.

6. Procédé selon l'une des revendications précédentes, le mélange réactionnel ne contenant pas de triéthanolamine, pas d'ions chlorure et/ou de sels de chlore, en particulier pas de NaCl.

7. Procédé selon l'une des revendications précédentes, le mélange réactionnel de l'étape v contenant 0,5 à 4 % en poids du complexe $Ag_2Zn(EDTA)$.

8. Procédé selon l'une des revendications précédentes, la somme des masses du prépolymère terminé par amine et du prépolymère terminé par isocyanate représentant 10 à 30 % en poids du mélange réactionnel.

9. Hydrogel doté de propriétés de réduction de biofilms, pouvant être obtenu par le procédé selon l'une des revendications précédentes.

10. Hydrogel selon la revendication 9, l'hydrogel présentant une teneur en eau de 40 à 65 % en poids.

11. Hydrogel selon la revendication 9 ou 10, l'hydrogel présentant une valeur du pH de 6,5 à 9,5, de préférence de 6,5 à 8.

**12.** Hydrogel selon l'une des revendications 9 à 11, l'hydrogel réduisant le nombre de cellule bactériennes de *S. aureus* ou de *P. aeruginosa* au sein d'un biofilm pendant un test selon la norme ASTM E2871-13 et pendant un temps de contact de 24 h d'au moins $\log_{10} = 2$.

**13.** Hydrogel selon l'une des revendications 9 à 12, l'hydrogel contenant 0,5 à 4 % en poids du complexe $Ag_2Zn(EDTA)$.

**14.** Hydrogel selon l'une des revendications 9 à 13, la libération d'humidité de l'hydrogel représentant au moins 5 mg par centimètre carré et par jour.

**15.** Pansement comprenant un hydrogel selon l'une des revendications 9 à 14 en tant que couche de contact avec la plaie, ainsi qu'une couche support opposée à la couche de contact avec la plaie, la couche support comprenant éventuellement une zone adhésive périphérique.

**16.** Pansement selon la revendication 15, comprenant en outre, entre la couche de contact avec la plaie et la couche support, une mousse absorbante, de préférence une mousse absorbante en polyuréthane.

Figur 1

Figur 2

Figur 3

0,5% Wirkstoffgehalt

Figur 4a

Figur 4b

Figur 4c

Figur 5

Figur 6

Figur 7

☐ S. aureus   ■ P. aeruginosa

Figur 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017191453 A

- WO 2010000450 A

**In der Beschreibung aufgeführte Nicht-Patentliteratur**

- **GRIP, JOSTEIN et al.** Sprayable Carbopol hydrogel with soluble beta-1, 3/1, 6-glucan as an active ingredient for wound healing-development and in-vivo evaluation.. *European Journal of Pharmaceutical Sciences*, 2017, vol. 107, 24-31